# EUROPEAN PATENT APPLICATION

(11) **EP 1 690 929 A1**
(43) Date of publication of application: **16.08.2006**
(21) Application number: 04792726.4
(22) Date of filing: 14.10.2004
(51) Int. Cl.: C12N 5/08, A61K 35/12

(54) **REGENERATION TREATMENT SYSTEM**

(30) Priority: 14.10.2003 JP 2003354413
(71) Applicant: Nishida, Kohji, Ibaraki-shi, Osaka 567-0031 (JP); Maeda, Kazuhisa, Takaishi-shi, Osaka 592-0002 (JP)
(72) Inventor: Nishida, Kohji, Ibaraki-shi, Osaka 567-0031 (JP); Maeda, Kazuhisa, Takaishi-shi, Osaka 592-0002 (JP)
(74) Representative: HOFFMANN EITLE
(86) International application number: PCT/JP2004/015576
(87) International publication number: WO 2005/035739

(57) **Abstract**

The present invention aims at providing a technology that allows cells to be used efficiently and effectively in regenerative medicine. Therefore, the present invention provide a method for preparing a transplant for regenerating an organ, tissue or cell of a subject, comprising the steps of: A) providing part of a desired organ, tissue or cell or a stem cell capable of differentiation thereinto; and B) culturing the part or the stem cell on a feeder cell comprising an adipose tissue derived cell. The present invention also provide a feeder cell, system and so on used in such a method.

## Description

### TECHNICAL FIELD

This invention relates to the field of regenerative medicine. More particularly, this invention relates to a technology for regenerating and maintaining organs, tissues and cells. This invention also provides a regenerative medical treatment method and a system that make use of the technology of the Invention.

### BACKGROUND ART

Research of regenerative medicine has recently seen rapid advances and its potential has been drawing much attention. Materials used in regenerative medicine are embryonic stem (ES) cells and it is held essential that ES cells be cultured on feeder cell layers such as that made of a mouse derived STO cell line or a primary cultured mouse fibroblasts prepared from fetal mouse. It is known that the primary cultured mouse cells supports stem cells of not only mouse but also other species as well.

Regenerative medicine is practiced in various fields and one of the projects that are currently most highlighted is corneal regenerative medicine. In the conventional human regenerative therapy, mouse fibroblasts which are xenogeneic cells need to be used *ex vivo* or *in vitro* and this has been a great barrier to clinical application of the technology. This is because cells from an animal of a different species must be used as feeder cells in the currently practiced corneal regenerative medicine. According to FDA directives, the transplanted tissue has to be clinically examined for 20 years but this is not a practical guideline if the technology is to be clinically applied in therapy.

It has become known that fat also contains stem cells (WO 03/022988 and WO 00/53795). Stem cells in adipose tissue, compared to those in other tissues (e.g. bone marrow) are rich in supply sources, with apparently high abundance, so their potential use is drawing increasing attention. However, no idea has been reported of using stem cells per se as the support or feeder in regenerative medicine.

For therapy in humans, it is preferred to use human derived cells or cell components but very few cases of success in using human derived cells as feeder cells have been reported. It has conventionally be held that a cell line established from human derived cells has only low efficiency as a feeder, so it is predicted that human derived cells cannot be used as effective feeder cells.

For example, US 2002-0072117 A which describes a cell line of human derived mesenchymal stem cells or fibroblasts as feeder cells for culturing human ES cells. Being a cell line differentiated and immortalized from pluripotent stem cells (ES cells), the cells do not appear to be malignant. However, efficiency of the feeder is low. US 2002-197717 A describes a feeder cell for culturing human hematopoietic precursor cells. However, this cell is a cell line of human stromal cell which, according to the patent application, is designated bone marrow stromal cell HS-5. This cell is said to secrete LIF, KL, MIPlalpha or IL-6. WO 95/02040 describes feeder cell L87/4 or L88/5 mainly used for culturing blood cells. These cells comprise a cell line established by exposing human bone marrow stromal cells to a radiation so that it becomes immortal and the irradiated bone marrow stroma cells have an increased expression of G-CSF or IL-6. US 2003-143736 A describes feeder cells used for culturing human ES cells. These feeder cells are human adult/fetal/embryonic cells or combinations thereof; the patent application also says that the feeder cells are fibroblasts/skin cells/fibroblasts in muscle tissue/epithelial cells or combinations thereof. However, all these cells are established and even seem to contain something like fallopian tube fibroblasts. In US 2003-143736 A, the feeder cells are established as primary cultures in HFE medium which are maintained and propagated in HM medium (Detroit 551, MRC-5 and WI-38).

Nevertheless, everyone in the art feels that human derived cells are preferably used as feeder cells or other essential components for regenerative therapy and there is a particular need to make available those human derived cells which can be used as feeder cells *ex vivo.*

With remarkable advances in medical technology, organ transplant has recently become a common clinical practice to replace a difficult to treat organ with another's organ. However, the number of donors still remains small and in the case of corneal transplantation, there are about twenty thousand patients annually in Japan who need corneal transplantation but only a tenth of them (about 2000) can receive grafting treatment. Although corneal transplantation is almost established in medicine, a technology of next generation is required on account of the shortage of donors.

Under these circumstances, the grafting technology of using either artificial substitutes or tissues formed by culturing cells has long been drawing attention. Typical examples of such technology include artificial skin and cultured skin. Artificial skin prepared from synthetic polymers can potentially cause side effects such as rejection and are not preferred as skin grafts. On the other hand, cultured skin is prepared by culturing part of the normal skin of a patient to a desired size, so in the absence of any concern such as rejection that may result from its use, the cultured skin may well be called the most natural masking agent.

JP H02-23191 B describes a process for producing a transplantable cultured cell membrane characterized by culturing human neonate derived epidermal keratinocytes under such conditions that a membrane of keratin tissue is formed on the surface of a vessel and peeling off the formed keratin tissue membrane using an enzyme. Specifically, the use of 3T3 cells as a feeder layer permits the seeded epithelial cells not only to grow but also to be subsequently layered. This process has now become the most popular method of culturing epidermal keratinocytes. However, this method has an often pointed out drawback, i.e. the 3T3 cells are mouse derived cells. It is generally said that the 3T3 cells will disappear in the process of culturing the epidermal keratinocytes but as of today, 100% disappearance of those cells has not been demonstrated.

Various proposals have so far been made with a view to solving this problem. Among them are a method comprising the steps of culturing 3T3 cells on a separate culture substrate so that a substance effective for epidermal keratinocytes will be released in the medium and then selectively transferring the conditioned medium into a system where epidermal keratinocytes are being cultured (JP 9-313172 A and JP 2001-149070 A). This method can prevent the contamination of cells from an animal of a different species; however, since various proteins produced by cells from an animal of a different species are not separated from each other, the method still involves basic problems similar to those described in the preceding paragraphs. Attempts have also been made to use human derived cells as feeder layers but in the absence of any cells produced to date that have comparable activity to that of the 3T3 cells, there has been a strong need for an effective technology that can replace the 3T3 cells. The conventional methods are summarized in "Protocols of Studies on Stem Cells and Clones", ed. by Nakatsuji, Yohdosha (2001) (in Japanese) but none of them is a real breakthrough.

JP 2004-248655 A focuses on culture media but the need for feeder cells still remains valid.

### DISCLOSURE OF THE INVENTION

Problem to be Solved: The present invention aims at providing a technology that allows cells to be used efficiently and effectively in regenerative medicine.

Means for Solving the Problem: The system of the present invention has been accomplished by finding the fact that adipose tissue derived cells can be utilized as feeder cells. In another aspect, the stated problem has been solved by human derived fibroblasts. In a preferred embodiment, the system of the present invention permits the use of autologous cells and may well be described as a therapeutic method that is extremely effective and finds a very wide scope of applications. Therefore, the present invention provides the following.
(1) An adipose tissue derived cell for use as a feeder cell.
(2) The cell according to item 1 wherein the feeder cell is for differentiating or maintaining an embryonic stem cell, a tissue stem cell or a differentiated cell.
(3) The cell according to item 1 wherein the feeder cell is for effecting differentiation to or maintenance of epidermis.
(4) The cell according to item 1 wherein the feeder cell is for effecting differentiation to or maintenance of cornea.
(5) The cell according to item 1 wherein the cell comprises a tissue stem cell.
(6) The cell according to item 1 wherein the cell comprises a fibroblast.
(7) The cell according to item 1 wherein the cell comprises a primary cultured cells.
(8) The cell according to item 1 wherein the cell comprises a human cell.
(9) A method for preparing a transplant for regenerating an organ, tissue or cell of a subject, comprising the steps of:
   A) providing part of a desired organ, tissue or cell or a stem cell capable of differentiation thereinto; and
   B) culturing the part or the stem cell on a feeder cell comprising an adipose tissue derived cell.
(10) The method according to item 9 wherein the desired organ, tissue or cell comprises an epidermic one.
(11) The method according to item 9 wherein the desired organ, tissue or cell is selected from the group consisting of cornea, bone, muscle, cartilage, heart, pericardium, blood vessel, skin, kidney, liver, umbilical cord, intestine, nerve, lung, placenta, pancreas, brain, joint, limb peripheries, fat, retina, and parts thereof.
(12) The method according to item 9 wherein the feeder cell comprises a tissue stem cell.
(13) The method according to item 9 wherein the feeder cell comprises a fibroblast.
(14) The method according to item 9 wherein the feeder cell comprises a primary cultured cells.
(15) The method according to item 9 wherein the subject and the feeder cell are derived from the same species.
(16) The method according to item 9 wherein the subject is human and the feeder cell is a human cell.
(17) The method according to item 9 wherein the culturing is performed *ex vivo.*
(18) The method according to item 9 wherein the part or the stem cell and the feeder cell are xenogeneic, allogeneic or syngeneic.
(19) The method according to item 9 wherein the part or the stem cell and the feeder cell are syngeneic.
(20) The method according to item 9 wherein the part or the stem cell is either what has been just excised from the subject or what has been stored frozen.
(21) The method according to item 9 wherein the culturing is performed in the presence of at least one factor selected from the group consisting of fetal calf serum, insulin or cholera toxin.
(22) The method according to item 9 wherein the culturing is performed at the ratio of the part or the stem cell to the feeder cell between the range of 10:1 to 1:10.
(23) The method according to item 9 wherein the culturing is performed using the feeder cell in a smaller amount than the part or the stem cell.
(24) The method according to item 9 wherein the culturing is performed in a culture medium containing a cytophysiologically active substance.
(25) The method according to item 9 wherein the culturing is performed in a culture medium containing an epidermal growth factor (EGF) and wherein the desired organ, tissue or cell contains cornea, its tissue or cell.
(26) The method according to item 9 which further includes the step of suppressing the growth of the feeder cell.
(27) The method according to item 26 wherein the suppressing of the growth of the feeder cell is achieved by an antibiotic administration or an irradiation.
(28) The method according to item 27 wherein the antibiotic comprises mitomycin C.
(29) A system for regenerating an organ, tissue or cell of a subject, comprising:
   A) a vessel; and
   B) a feeder cell comprising an adipose tissue derived cell.
(30) The system according to item 29 which further comprises a providing means for providing part of a desired organ, tissue or cell or a stem capable of differentiation thereinto.
(31) The system according to item 30 wherein the providing means includes a means for recovering the part or the stem cell from the subject.
(32) The system according to item. 31 wherein the means includes means selected from the group consisting of a catheter, a scraping rod, forceps, a syringe, medical scissors, and an endoscope.
(33) The system according to item 29 which further comprises a cytophysiologically active substance.
(34) The system according to item 29 which further comprises EGF.
(35) The system according to item 29 which further includes means for suppressing the growth of the feeder cell.
(36) A method for regenerating an organ, tissue or cell of a subject, comprising the steps of:
   A) providing part of a desired organ, tissue or cell or a stem cell capable of differentiation thereinto;
   B) culturing the part or the stem cell on a feeder cell comprising an adipose tissue derived cell; and
   C) transplanting the cultured part or stem cell to a site to be treated of the subject.
(37) A method for regenerating an organ, tissue or cell of a subject, comprising the step of:
   A) transplanting part of a desired organ, tissue or cell or a stem cell capable of differentiation thereinto and a feeder cell comprising an adipose tissue derived cell to a site to be treated of the subject.
(38) A pharmaceutical composition for regenerating an organ, tissue or cell of a subject, comprising:
   A) part of a desired organ, tissue or cell or a stem cell capable of differentiation thereinto and a feeder cell comprising an adipose tissue derived cell.
(39) Use of an adipose tissue derived cell as a feeder cell.
(40) Use of an adipose tissue derived cell for producing a pharmaceutical composition containing a feeder cell.
(41) Use of an adipose tissue derived cell for producing a pharmaceutical composition for regenerating an organ, tissue or cell of a subject.
(42) A primary cultured human fibroblasts for use as a feeder cell.
(43) A method for preparing a transplant for regenerating an organ, tissue or cell of a subject, comprising the steps of:
   A) providing part of a desired organ, tissue or cell or a stem cell capable of differentiation thereinto; and
   B) culturing the part or the stem cell on a feeder cell comprising a primary cultured human fibroblasts.
(44) A system for regenerating an organ, tissue or cell of a subject, comprising:
   A) a vessel; and
   B) a feeder cell comprising a primary cultured human fibroblasts.
(45) A method for regenerating an organ, tissue or cell of a subject, comprising the steps of:
   A) providing part of a desired organ, tissue or cell or a stem cell capable of differentiation thereinto;
   B) culturing the part or the stem cell on a feeder cell comprising a primary cultured human fibroblasts; and
   C) transplanting the cultured part or stem cell to a site to be treated of the subject.
(46) A method for regenerating an organ, tissue or cell of a subject, comprising the step of:
   A) transplanting part of a desired organ, tissue or cell or a stem cell capable of differentiation thereinto and a feeder cell comprising a primary cultured human fibroblasts to a site to be treated of the subject.
(47) A pharmaceutical composition for regenerating an organ, tissue or cell of a subject, comprising:
   A) part of a desired organ, tissue or cell or a stem cell capable of differentiation thereinto and a feeder cell comprising a primary cultured human fibroblasts.
(48) Use of a primary cultured human fibroblasts as a feeder cell.
(49) Use of a primary cultured human fibroblasts for producing a pharmaceutical composition containing a feeder cell.
(50) Use of a primary cultured human fibroblasts for producing a pharmaceutical composition for regenerating an organ, tissue or cell of a subject.
(51) A graft for regenerating an epithelial tissue which contains a stem cell or a cell derived from the stem cell.
(52) The graft according to item 51 wherein the epithelial tissue is cornea.
(53) The graft according to item 51 wherein the stem cell is selected from the group consisting of an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell and a hepatic stem cell.
(54) The graft according to item 51 wherein the stem cell is an epithelial stem cell.
(55) The graft according to item 51 wherein the stem cell is selected from the group consisting of a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, and a gallbladder epithelial stem cell.
(56) The graft according to item 51 wherein the stem cell or the cell derived from the stem cell has been co-cultured with a feeder cell.
(57) The graft according to item 56 wherein a human derived cell is used as the feeder cell.
(58) The graft according to item 57 wherein the human derived cell comprises an adipose derived cell, an embryonic stem cell or a bone marrow stem cell.
(59) The graft according to item 58 wherein a cell cultured in DMEM+10% FBS in the absence of the feeder cell is used as the embryonic stem cell or bone marrow stem cell.
(60) The graft according to item 56 wherein the feeder cell comprises an adipose tissue derived cell.
(61) The graft according to item 56 wherein co-culturing with the feeder cell is performed under conditions that promote cell adhesion.
(62) The graft according to item 51 which comprises layered cells.
(63) The graft according to item 51 which is used in non-suture transplantation.
(64) The graft according to item 51 which does not contain any xenogeneic component.
(65) Use for preparing a pharmaceutical composition as a graft for regenerating an epithelial tissue, the graft containing a stem cell or a cell derived from the stem cell.
(66) The use according to item 65 wherein the epithelial tissue is cornea.
(67) The use according to item 65 wherein the stem cell is selected from the group consisting of an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell and a hepatic stem cell.
(68) The use according to item 65 wherein the stem cell is an epithelial stem cell.
(69) The use according to item 65 wherein the stem cell is selected from the group consisting of a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, and a gallbladder epithelial stem cell.
(70) The use according to item 65 wherein the stem cell or the cell derived from the stem cell has been co-cultured with a feeder cell.

Therefore, these and other advantages of the present invention will become apparent to skilled artisans by reading and understanding the following detailed description with reference to the attached drawings.

Advantageous Effects of the Invention: A system of regenerative therapy is provided using abundantly available sources. This is an advantageous effect that has so far been unattainable. In a preferred embodiment of the present invention, autologous cornea is cultured *ex vivo* and transplanted. Therefore, in this preferred embodiment, the system permits effective and simple use of autologous cells, thus eliminating the concerns heretofore talked about (e.g. risk of infectious disease). This feature, combined with the total absence of immunological rejection, contributes to remarkably widening the scope of applications of the conventional technology of regenerative medicine. Cell sources for corneal regenerative medicine have quite a lot of needs to meet in ethical and social viewpoints. Furthermore, in the case of using a primary cultured human fibroblasts, the system of the present invention can meet the FDA's directives in cell therapy, thus offering immeasurable advantages.

### BRIEF DESCRIPTION OF THE DRAWINGS

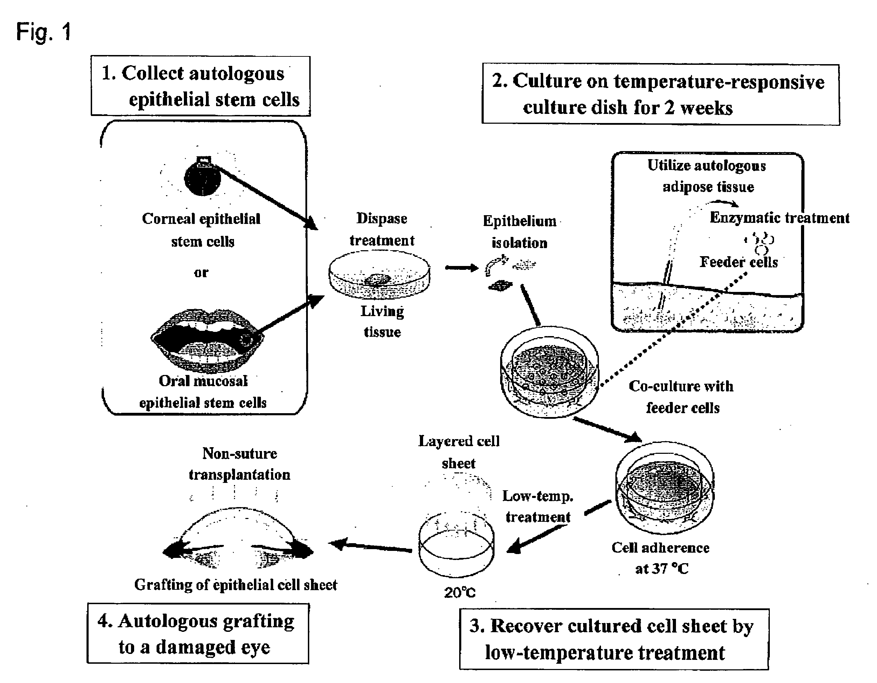

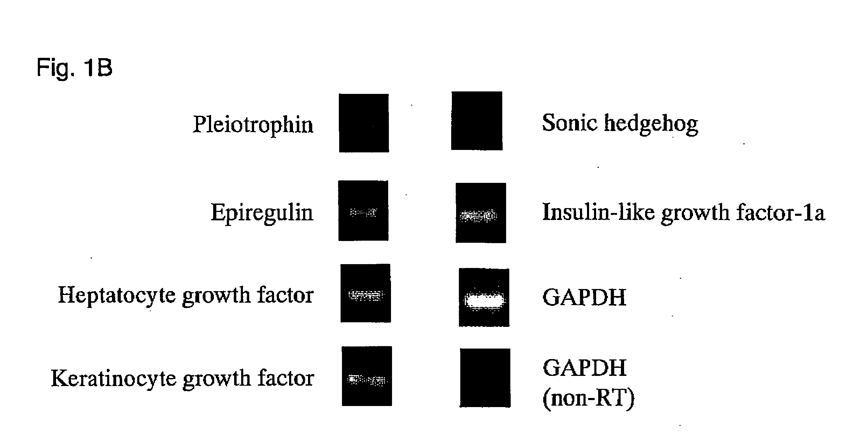

Fig. 1 is a schematic diagram showing how a method of corneal regenerative medicine by grafting a cultured cell sheet was developed; it shows corneal regenerative medicine using adipose tissue derived feeder cells.
Fig. 1B shows mRNA expression of adipose precursor cells; the expression of mRNA in adipose precursor cells was analyzed for proteins and the like that are assumed to affect the growth and maintenance of epithelial cells on NIH/3T3; in particular, cystatin C, hepatocyte growth factor (HGF), keratinocyte growth factor (KGF), insulin-like growth factor (IGF)-1a were expressed, and it is assumed that adipose precursor cells can be used as feeder cells for epithelial cells.

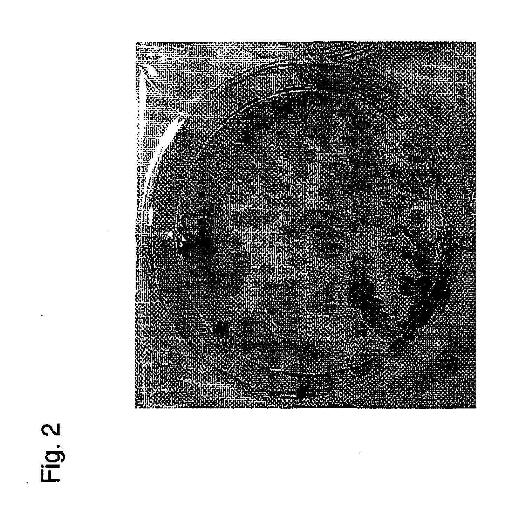

Fig. 2 shows the appearance of corneal epithelial stem cells on 3T3 feeder cells (2 x 10⁴ cells/cm²) used as a control in Example 6.

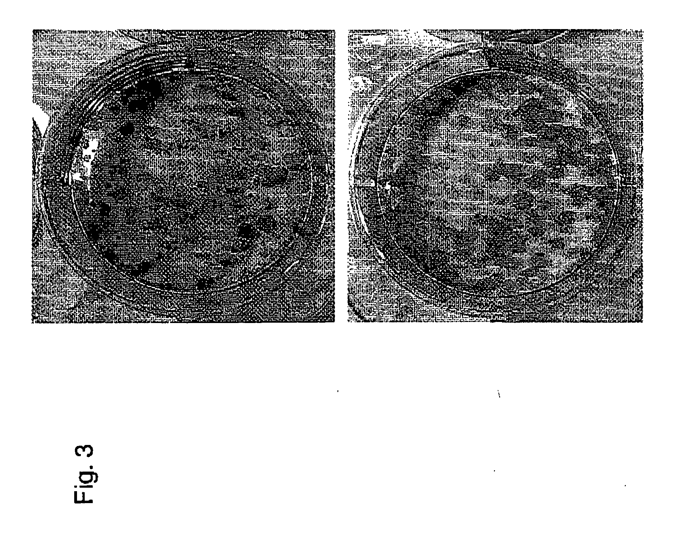

Fig. 3 shows the appearance of corneal epithelial stem cells on the feeder cells of the present invention (derived from the deep tissue; (1 x 10⁴ cells/cm²)) used in Example 6; the upper and lower panels show the difference in the concentration of mitomycin (upper panel: 40 µg/ml; lower panel: 80 µg/ml).

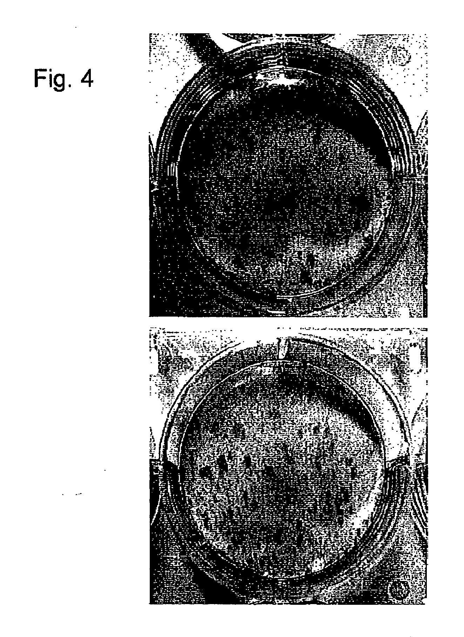

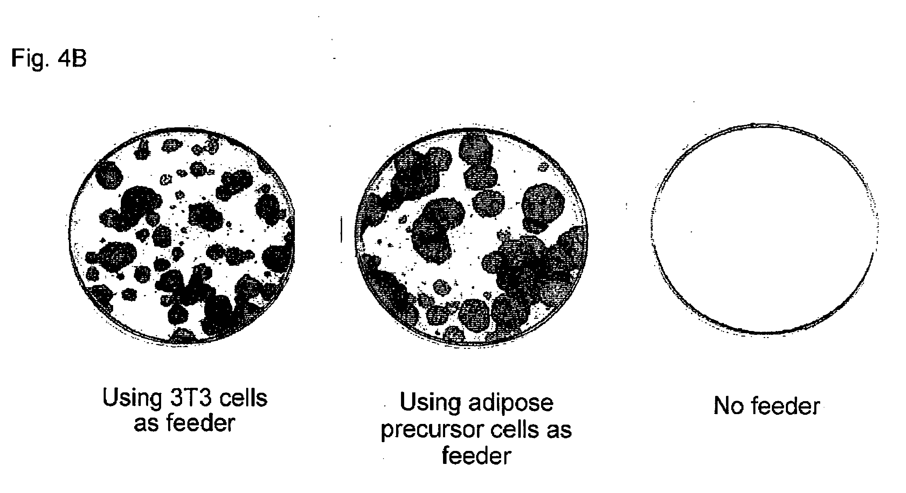

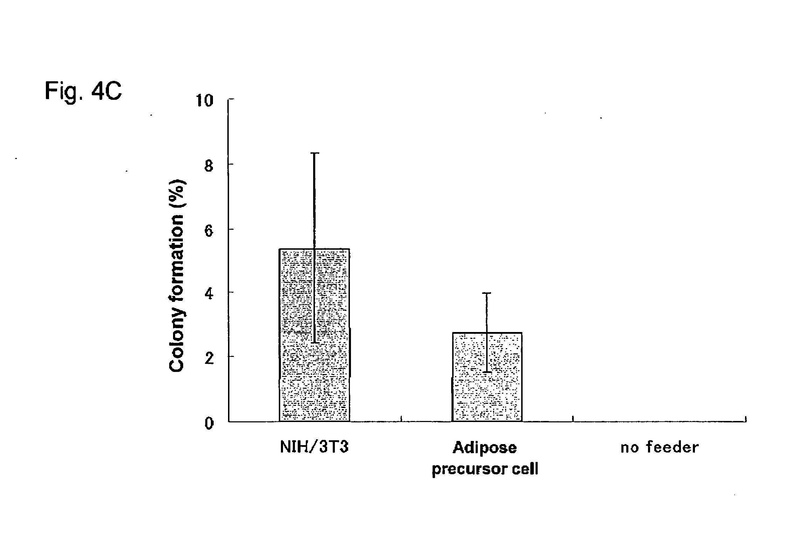

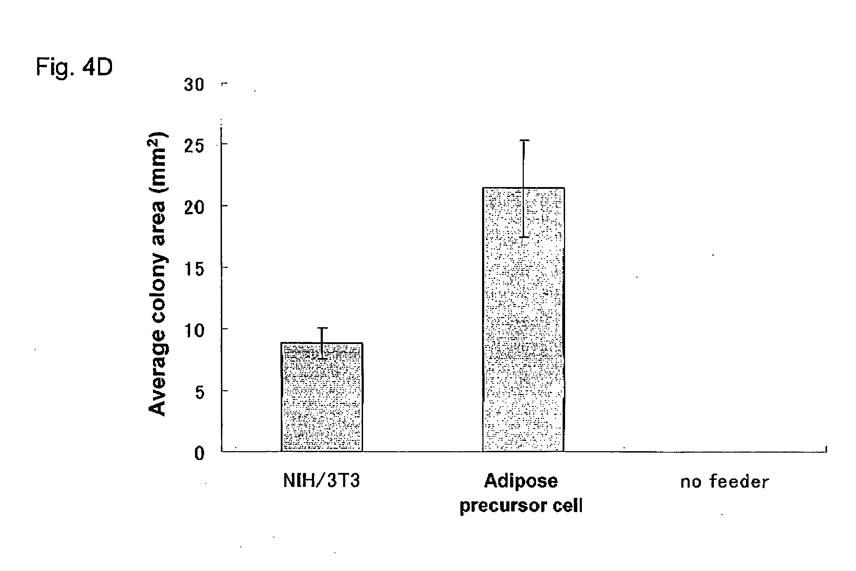

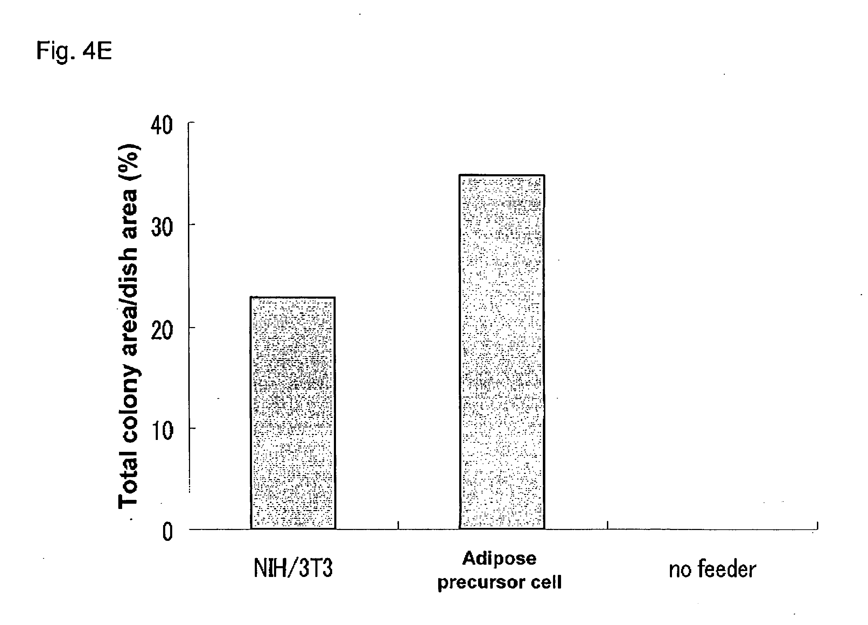

Fig. 4 shows the appearance of corneal epithelial stem cells on the feeder cells of the present invention (derived from the subcutaneous tissue; (1 x 10⁴ cells/cm²)) used in Example 6; the upper and lower panels show the difference in the concentration of mitomycin (upper panel: 16 µg/ml; lower panel: 16 µg/ml).
Fig. 4B shows an assay of human corneal epithelial cell colonies; it shows the feeder effect of human adipose tissue derived cells on human corneal epithelial stem cells; after irradiating NIH/3T3 and adipose precursor cells with X-rays (20 Gy), one-day culture was effected, followed by trypsin treatment to prepare feeder cells; then human corneal epithelial cells were seeded at a density of 1000 cells/dish; at 14 days of the culture, the cells were fixed by formalin fixation, and then stained by Rhodamine B; compared with NIH/3T3 feeder, the adipose precursor cell feeder enabled larger colonies to be observed.
Fig. 4C shows the percent colony formation; it shows the feeder effect of human adipose tissue derived cells on human corneal epithelial stem cells; comparison of the percent colony formation was made for n=6; among colonies formed by culture conducted on NIH/3T3 feeder and the adipose precursor cell feeder, the number of colonies larger than 5 mm in diameter was counted visually; the percent colony formation was calculated, with the number of seeded cells (1000) put as a denominator and the number of >5 mm colonies as a numerator; the percent colony formation from NIH/3T3 feeder was about twice that from the adipose precursor cell feeder.
Fig. 4D shows the average area of colonies; it shows the feeder effect of human adipose tissue derived cells on human corneal epithelial stem cells; comparison of the average area of colonies was made for n=6; the size of colonies formed by culture conducted on NIH/3T3 feeder and the adipose precursor cell feeder was measured using NIH image; the average colony size from the adipose feeder was about 2.4 times as large as from NIH/3T3.
Fig. 4E shows the percentage of the overall dish area occupied by the area of colonies; it shows the feeder effect of human adipose tissue derived cells on human corneal epithelial stem cells; data was calculated from Figs. 4C and 4D; the percentage of the overall dish area occupied by the area of colonies is shown for n=6; the proportion of the dish area occupied by the area of colonies (total colony area/dish area) was calculated; since the size of Individual colonies was larger in the case of the adipose precursor cell feeder, the percentage of the dish occupied by the area of colonies from the adipose precursor cell feeder was about 1.5 times the value from NIH/3T3 feeder.

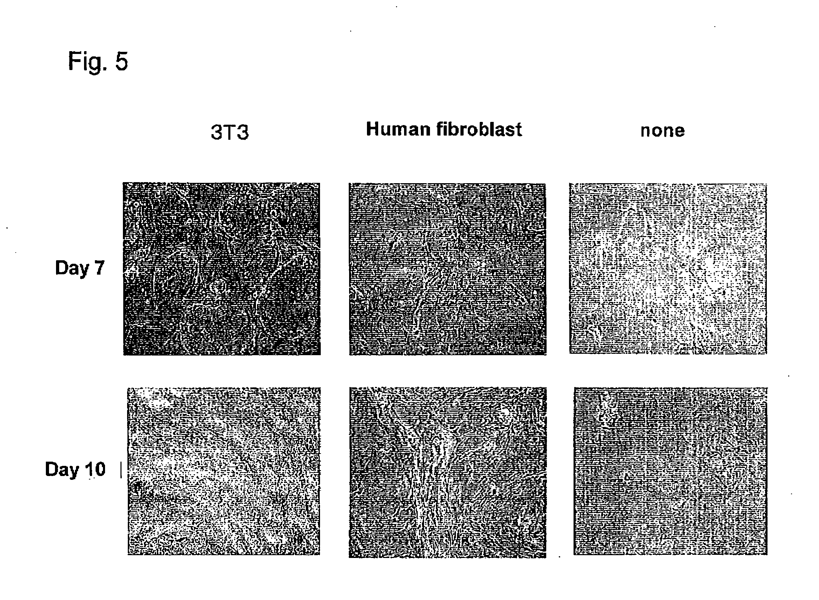

Fig. 5 is a set of micrographs demonstrating the effect exhibited by the feeder cells of the present invention in Example 9 to form a sheet of stem cells; human corneal epithelial cells were cultured at a density of 1 x 10⁵ cells/35 mm dish coated with N-isopropylacrylamide (NIPAAm): shown on the left are 3T3 cells (2 x 10⁴ cells/cm²); shown at the center are primary cultured human fibroblasts (2 x 10⁴ cells/cm²); shown on the right is a control (no feeder cells); the upper panels show the result at 7 days from the start of culture and the lower panels show the result at 10 days from the start of culture.

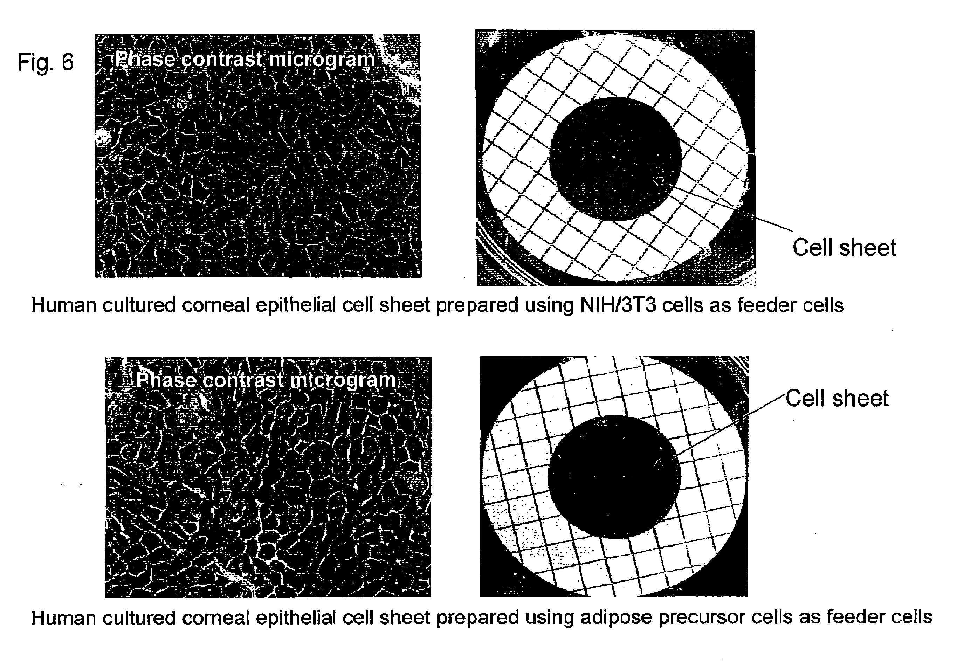

Fig. 6 shows examples of preparing corneal epithelial cell sheets; a sheet of human cultured corneal epithelial cells prepared using NIH/3T3 cells as feeder cells was compared with one prepared using adipose precursor cells as feeder cells; the upper panels show the case of using NIH/3T3 cells as feeder cells and the lower panels show the case of using adipose precursor cells as feeder cells; X-ray treated NIH/3T3 and adipose precursor cells were used as feeders in culturing human corneal epithelial cells; 35 mm dishes (coated with NIPAAm) were seeded with corneal epithelial cells at a density of 1 x 10⁵ cells/dish, which were peeled off at 14 days of culture to recover corneal epithelial cell sheets; the corneal epithelial cells cultured using the adipose precursor cell feeder formed a checkerboard pattern which was by no means inferior to the corneal epithelial cells cultured using the NIH/3T3 feeder.

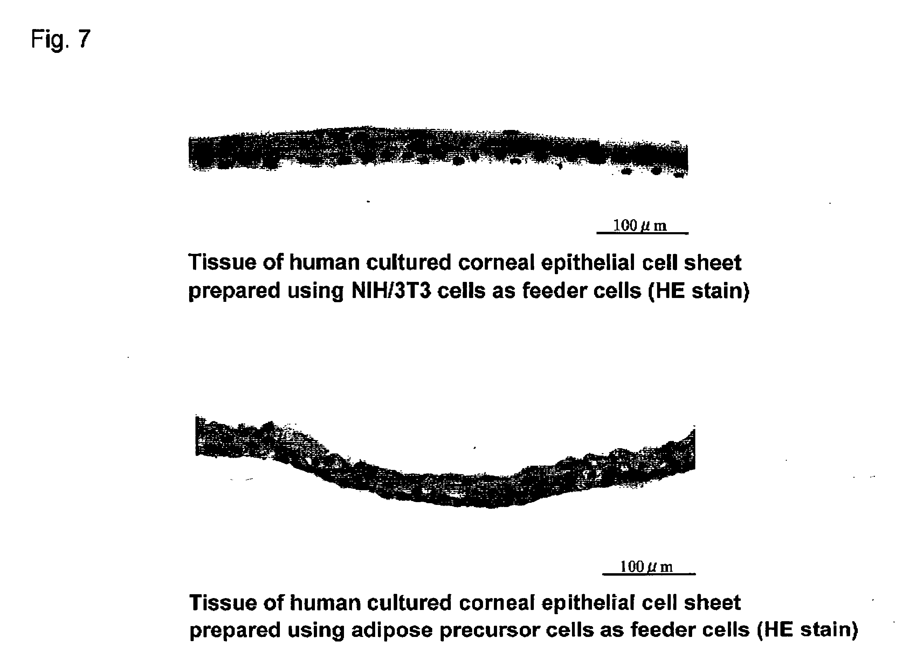

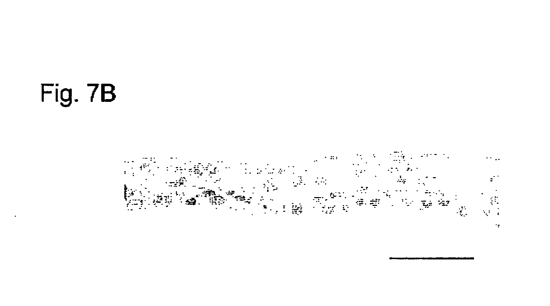

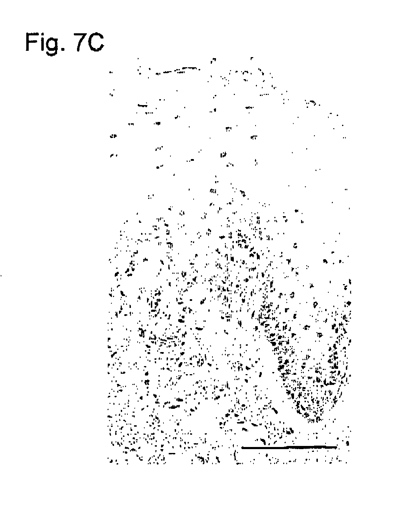

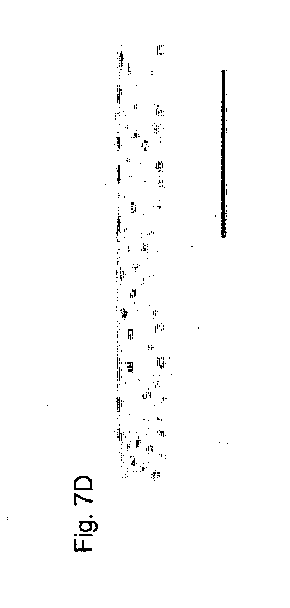

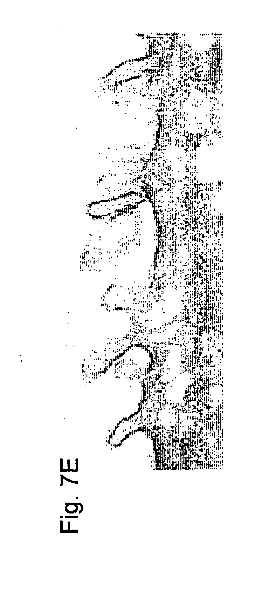

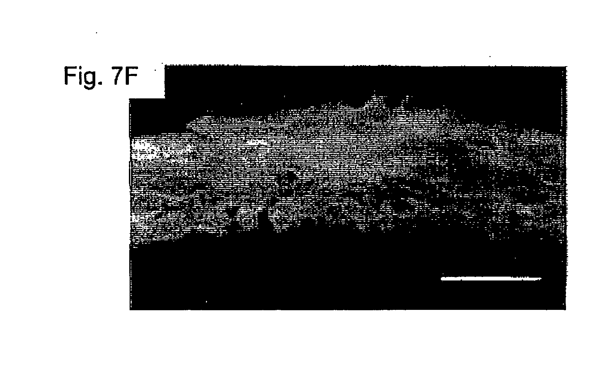

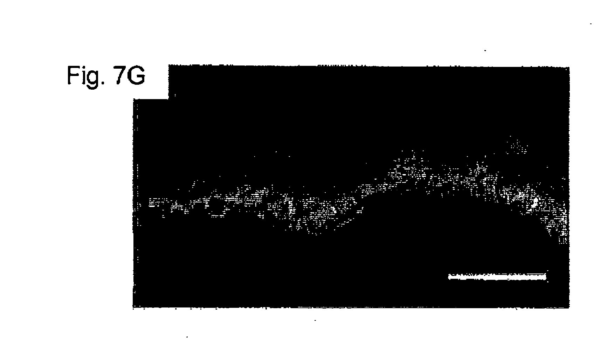

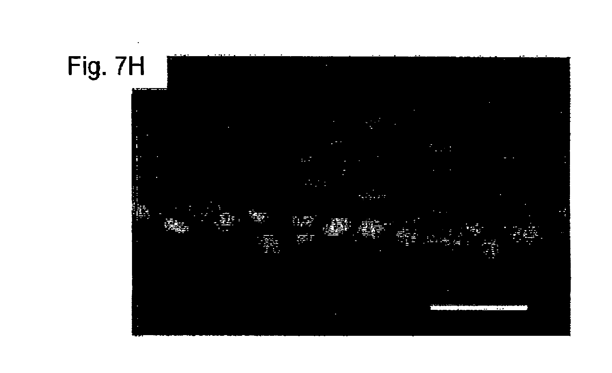

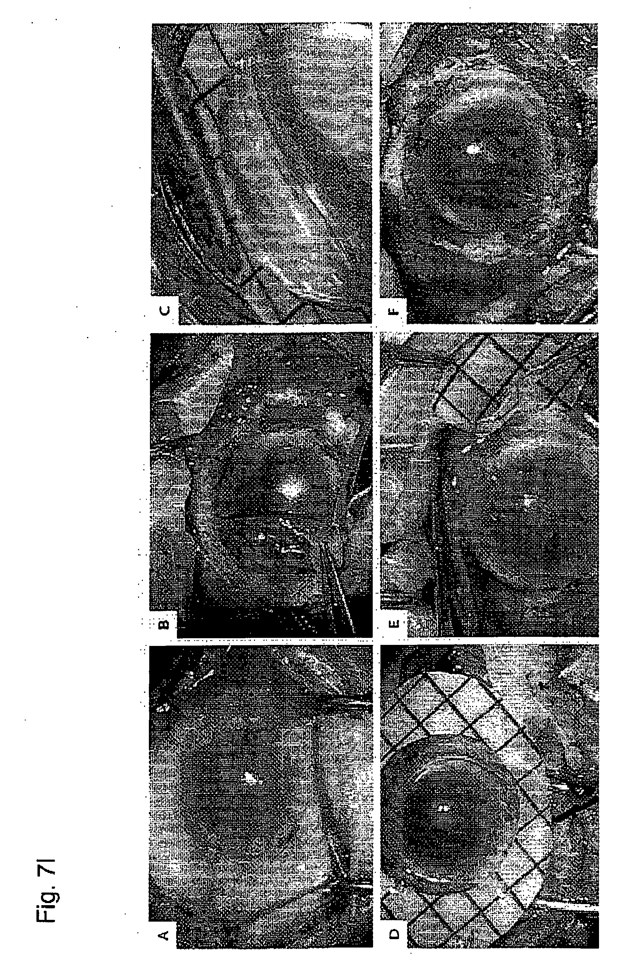

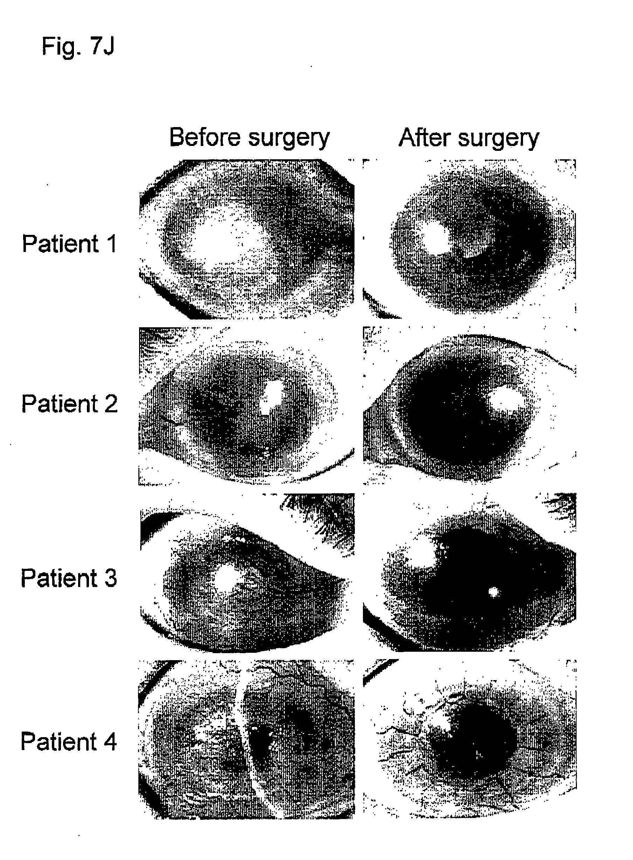

Fig. 7 shows examples of HE staining of corneal epithelial cell sheets; a sheet of human cultured corneal epithelial cells prepared using NIH/3T3 cells as feeder cells was compared with one prepared using adipose precursor cells as feeder cells; the upper panel shows the case of using NIH/3T3 cells as feeder cells and the lower panel shows the case of using adipose precursor cells as feeder cells; each of the corneal epithelial sheets prepared with NIH/3T3 and adipose precursor cell feeders consisted of 3-4 layers of epithelial cells, with comparatively small basement cells being observed at the basement and squamous epithelial cells in the upper layers: the corneal epithelial cell sheet prepared using the adipose precursor cell feeder was almost comparable to the sheet prepared using the NIH/3T3 feeder.
Fig. 7B shows the confirmation by H&E staining that the recovered cell sheet was formed of 3-5 cell layers.
Fig. 7C is an H&E stain of the initial oral mucosa which is entirely different from the cell sheet.
Fig. 7D shows the normal corneal epithelial cells; the cell sheet of the invention is similar to these corneal epithelial cells.
Fig. 7E is a micrograph showing the development of microvilli on the tip surface of the cell sheet to be used in the present invention.
Fig. 7F is a micrograph the cell sheet to be used in the present invention which was immunostained (in green) with anti-keratin 3 antibody.
Fig. 7G is a micrograph the cell sheet to be used in the present invention which was immunostained (in green) with anti-betal integrin antibody.
Fig. 7H is a micrograph the cell sheet to be used in the present invention which was immunostained (in green) with anti-p63 antibody.
Fig. 7I shows the process of transplanting the graft of the present invention onto cornea; Fig. 7I-A shows the overall surface of the cornea, with some angiogenesis occurring in the conjunctiva; Fig. 7I-B shows the conjunctival tissue on the cornea, which was re-exposed to reveal the corneal mesenchyme on the surface; Fig. 7I-C shows how the cell sheet is recovered by means of a doughnut-shaped supporter; Fig. 7I-D shows the cell sheet placed on the corneal mesenchyme; Fig. 7I-E shows the cell sheet that adhered in a few minutes to the corneal mesenchyme, from which the supporter was later detached; Fig. 7I-F shows engraftment of the cell sheet on the corneal mesenchyme.
Fig. 7J shows the appearance of the eyes of four patients after surgical operation; Figs. 7J-1 to 7J-4 refer to the respective patients; numerals 1 to 4 identify the patient numbers; the left panels show the pre-operative eyes and the right panels show the post-operative eyes.

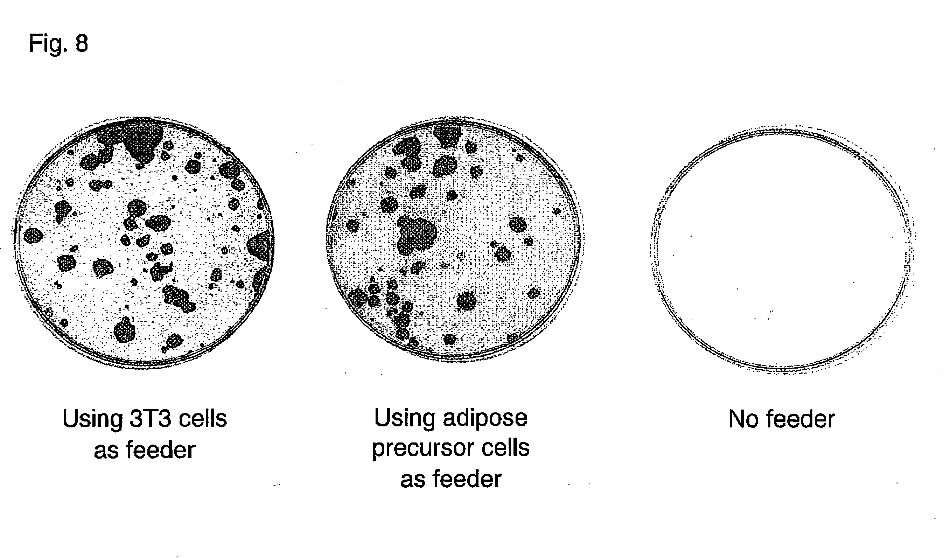

Fig. 8 shows an assay of human keratinocyte colonies which tests the feeder effect of adipose precursor cells: the feeder effect of adipose precursor cells on human keratinocytes was verified by the colony assay; after irradiating NIH/3T3 and adipose precursor cells with X-rays (20 Gy), one-day culture was effected, followed by trypsin treatment to prepare feeder cells; then human keratinocytes were seeded at a density of 1 x 10⁴ cells/dish; at 14 days of the culture, the cells were fixed by formalin, and then stained by Rhodamine B; the adipose precursor cells also showed the feeder effect on the keratinocytes, suggesting the possibility of their working as feeder cells for other epithelial cells than corneal epithelial cells.

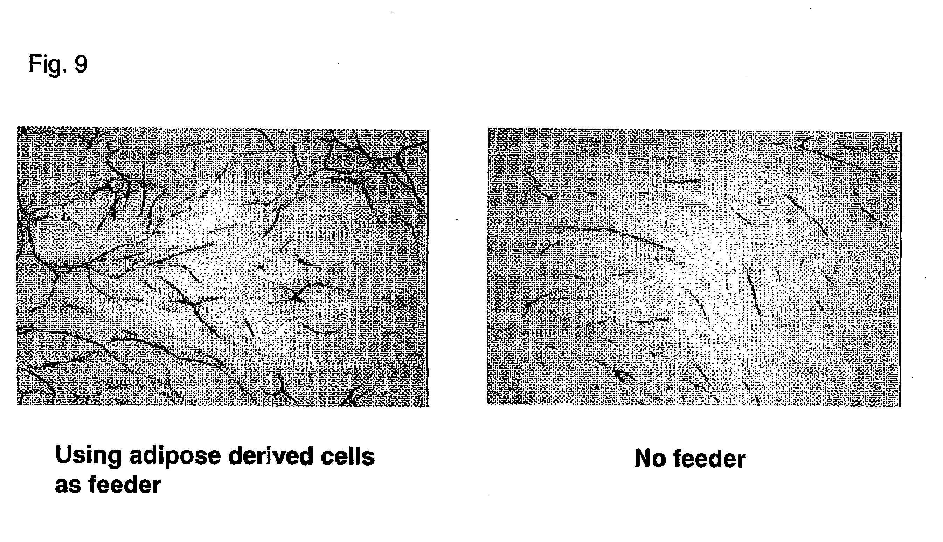

Fig. 9 shows the feeder effect of adipose precursor cells on human vascular endothelial cells; the feeder effect of adipose precursor cells on human vascular endothelial cells was verified as vessel formation; in order to see if human adipose precursor cells would become feeder cells capable of promoting blood vessel formation, the conditioned medium of the adipose precursor cells was added to a 3-dimensional co-culture system of human vascular endothelial cells and fibroblasts (Kurabo), whereupon the lumen forming capability was significantly enhanced in comparison with the conditioned medium of endothelial cells; discussing the migrating capability of endothelial cells, the conditioned medium of adipose precursor cells showed significant enhancement in comparison with the conditioned medium of endothelial cells; it was therefore clear that the adipose precursor cells also showed the feeder effect on the human vascular endothelial cells; Angiogenesis kit (Kurabo. Tokyo, Japan) was used.

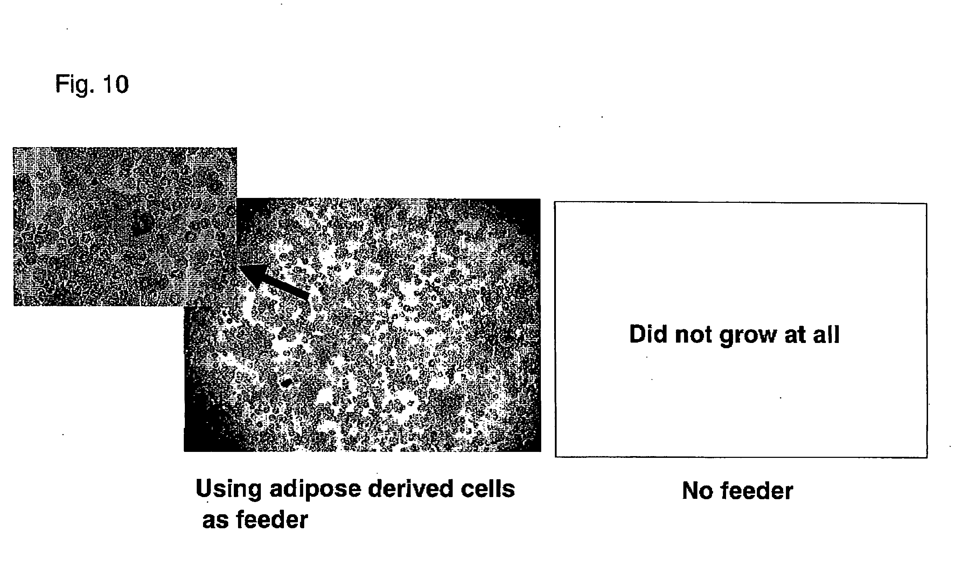

Fig. 10 shows the feeder effect of adipose precursor cells on human bone marrow derived mesenchymal stem cells (MNC); the feeder effect of adipose precursor cells on human bone marrow derived mesenchymal stem cells was verified; comparison was made between a culture medium alone (10% FCS + DMEM) and the culture medium with mouse adipose precursor cells as feeder cells; bone marrow mononuclear cells were isolated from mouse bone marrow by Histopaque (density-gradient centrifugation method) and seeded on both samples; at 7 days of the culture, the suspended cells were separated and subjected to May-Giemsa staining; it became clear that the adipose precursor cells also showed the feeder effect on the bone marrow mononuclear cells .

### DESCRIPTION OF THE SEQUENCT LISTING

SEQ ID NO: 1 shows the sense primer for detecting pleiotrophin.
SEQ ID NO: 2 shows the antisense primer for detecting pleiotrophin.
SEQ ID NO: 3 shows the sense primer for detecting epiregulin.
SEQ ID NO: 4 shows the antisense primer for detecting epiregulin.
SEQ ID NO: 5 shows the sense primer for detecting a hepatocyte growth factor.
SEQ ID NO: 6 shows the antisense primer for detecting a hepatocyte growth factor.
SEQ ID NO: 7 shows the sense primer for detecting a keratinocyte growth factor.
SEQ ID NO: 8 shows the antisense primer for detecting a keratinocyte growth factor.
SEQ ID NO: 9 shows the sense primer for detecting sonic hedgehog.
SEQ ID NO: 10 shows the antisense primer for detecting sonic hedgehog.
SEQ ID NO: 11 shows the sense primer for detecting insulin-like growth factor 1a.
SEQ ID NO: 12 shows the antisense primer for detecting insulin-like growth factor 1a.
SEQ ID NO: 13 shows the sense primer for detecting glyceraldehyde 3-phosphate dehydrogenase (GAPDH).
SEQ ID NO: 14 shows the antisense primer for detecting GAPDH.
SEQ ID NO: 15 shows the sense primer for cystatin C.
SEQ ID NO: 16 shows the antisense primer for cystatin C.

**SEQUENCE DATA AND PRODUCT SIZE**

| Gene's name | Sequence (5'-3') | Product size (bp) |
|---|---|---|
| Pleiotrophin (sense) (SEQ ID NO: 1) | AGAGGACGTTTCCAACTCAA | 551 |
| Pleiotrophin (antisense) (SEQ ID NO: 2) | TATGTTCCACAGGTGACATC | |
| Epiregulin (sense) (SEQ ID NO: 3) | AGGAGGATGGAGATGCTCTG | 497 |
| Epiregulin (antisense) (SEQ ID NO: 4) | TCAGACTTGCGGCAACTCTG | |
| Hepatocyte growth factor (sense) (SEQ ID NO: 5) | GCCTGAAAGATATCCCGACA | 522 |
| Hepatocyte growth factor (antisense) (SEQ ID NO: 6) | TTCCATGTTCTTGTCCCACA | |
| Keratinocyte growth factor (sense) (SEQ ID NO: 7) | GGAGGAATCCTGTGTTGTTA | 504 |
| Keratinocyte growth factor (antisense) (SEQ ID NO: 8) | CTAAGGTTCATGAGCAGTAC | |
| Sonic hedgehog (sense) (SEQ ID NO: 9) | CGGAGCGAGGAAGGGAAAG | 261 |
| Sonic hedgehog (antisense) (SEQ ID NO: 10) | TTGGGGATAAACTGCTTGTAGGC | |
| Insulin-like growth factor la (sense) (SEQ ID NO: 11) | ATGCACACCATGTCCTC | 389 |
| Insulin-like growth factor la (antisense) (SEQ ID NO: 12) | CATCCTGTAGTTCTTGTTTC | |
| GAPDH (sense) (SEQ ID NO: 13) | ACCACAGTCCATGCCATCAC | 451 |
| GAPDH (antisense) (SEQ ID NO: 14) | TCCACCACCCTGTTGCTGTA | |
| Cystatin C (sense) (SEQ ID NO: 15) | TCCTCTCTATCTAGCTCCAG | 499 |
| Cystatin C (antisense) (SEQ ID NO: 16) | TCCTGACAGGTGGATTTCGA | |

### BEST MODE FOR CARRYING OUT THE INVENTION

The present invention will be described below. It should be understood throughout the present specification that articles for singular forms include the concept of their plurality unless otherwise mentioned. Therefore, articles for singular forms (e.g., "a", "an", "the", and the like in English) include the concept of their plurality unless otherwise specified. It should be also understood that terms as used herein have definitions ordinarily used in the art unless otherwise mentioned. Therefore, all technical and scientific terms used herein have the same meanings as commonly understood by those skilled in the relevant art. Otherwise, the present specification (including definitions) takes precedence.

### (Definition of terms)

The definitions of specific terms used herein are described below.

As used herein, the term "organ" refers to a structure which is a specific part of an individual organism where a certain function of the individual organism is locally performed and which is morphologically independent. Generally, in multicellular organisms (e.g., animals and plants), organs are made of several tissues in specific spatial arrangement and tissue is made of a number of cells. Examples of such organs include, but are not limited to, skin, blood vessel, cornea, kidney, heart, liver, umbilical cord, intestine, nerve, lung, placenta, pancreas, brain, joint, bone, cartilage, peripheral limbs, retina, and the like. Examples of such organs include, but are not limited to, organs of the epidermal system, the pancreatic parenchymal system, the pancreatic duct system, the hepatic system, the blood system, the myocardial system, the skeletal muscle system, the osteoblast system, the skeletal myoblast system, the nervous system, the vascular endothelial system, the pigment system, the smooth muscle system, the fat system, the bone system, the cartilage system, and the like.

As used herein, the term "immune reaction" refers to a reaction due to the dysfunction of immunological tolerance between a graft and a host. Examples of immune reactions include, but are not limited to, a hyperacute rejection reaction (within several minutes after implantation) (immune reaction caused by antibodies, such as □-Gal or the like), an acute rejection reaction (reaction caused by cellular immunity about 7 to 21 days after implantation), a chronic rejection reaction (rejection reaction caused by cellular immunity 3 or more months after implantation), and the like.

As used herein, the elicitation of an immune reaction can be confirmed by histopathological examination of the type, number, or the like of infiltration of (immunological) cells into implanted tissue using staining (e.g., HE staining, etc.), immunological staining, or microscopic inspection of tissue sections.

As used herein, the term "tissue" refers to a cellular population having the same function and morphology in cellular organisms. In multicellular organisms, constituent cells are usually differentiated so that the cells have specialized functions, resulting in functional specialization. Therefore, multicellular organisms are not simple cell clusters, but constitute organic or social cellular population having a certain function and structure. Examples of tissues include, but are not limited to, integument tissue, connective tissue, muscular tissue, neuronal tissue, and the like. Tissue targeted by the present invention may be derived from any organ or part of an organism. In a preferred embodiment of the present invention, tissue targeted by the present invention includes, but is not limited to, blood vessels, blood vessel-like tissue, cardiac valves, pericardia, dura mater, cornea, joints, cartilage and bones. In the most preferred embodiment, tissue targeted by the present invention is cornea.

As used herein, the term "adipose tissue" collectively refers to fat storage tissues and includes areolar connective tissues having particularly high content of adipose cells, as exemplified by subcutaneous adipose tissue. Each adipose cell is surrouned by lattice fibers, with capillary blood vessels being densely distributed between cells, often forming fat bodies. In this specification, any kind of adipose tissue can be used as a source. The fat body is a mass or cluster of adipose tissue with substantially constant shapes which is independent of other tissues and in vertebrates it exists within the peritoneal cavity in contact with the kidney or gonad. The fat body often assumes a white, yellow or orange color.

As used herein, the term "membranous tissue" refers to a tissue in the form of membrane and is also referred to as "planar tissue". Examples of membranous tissue include a portion of tissue having a certain area of an organ (e.g., pericardium, dura mater, cornea, etc.) or bag-shaped tissue, and the like.

As used herein, the terms "implant", "graft", and "tissue graft" are used interchangeably, referring to homologous or heterologous tissue or a cellular population, or an artificial material, which is inserted into a particular site of a body and thereafter forms a part of the body. Therefore, the cultured cell of the present invention can be used as an implant. Examples of grafts include, but are not limited to, organs or portions thereof, cornea, blood vessels, blood vessel-like tissue, heart, cardiac valves, pericardia, and the like. Therefore, grafts encompass any one of those which are inserted into or substituted for a deficient or damaged part so as to compensate for the deficiency or damage. Grafts include, but are not limited to, autografts, allografts, and xenografts, which depend on the type of their donor.

The term "cell" is herein used in its broadest sense in the art, referring to a structural unit of tissue of a multicellular organism, which is a living body capable of self replicating, has genetic information and a mechanism for expressing it, and is surrounded by a membrane structure which separates the cell from the outside. In the method of the present invention, any cell can be used as a subject. The number of "cells" used in the present invention can be counted through an optical microscope. When counting the number of the cells using an optical microscope, the number of nuclei is counted. Tissues are sliced into tissue sections, which are then stained with hematoxylin-eosin (HE) to discriminate nuclei of cells from extracellular matrices (e.g., elastin or collagen). These tissue sections are observed under an optical microscope and the number of nuclei in a particular area (e.g., 200 µm x 200 µm) can be estimated to be the number of cells. Cells used herein may be either naturally occurring cells or artificially modified cells (e.g., fusion cells, genetically modified cells, etc.). Examples of cell sources may include, but are not limited to, a single-cell culture: the embryo, blood, or body tissue, or parts thereof (e.g. parts of oral mucosa and cornea) of a normally grown transgenic animal; a cell mixture of cells derived from normally grown cell lines: and the like. Alternatively, these sources can also be used as cells.

The cells to be used in the present invention may derive from any organisms as long as they have adipose cells or their counterparts (e.g.. Myxiniformes, Petromyzontiformes, Chondrichthyes, Osteichthyes, amphibian, reptilian, avian, mammalian, etc.). It is preferred that cells are derived from mammalians (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). In one embodiment, cells derived from primates (e.g., chimpanzee, Japanese monkey, human, etc.) are used; in the most preferred embodiment, cells derived from humans are particularly used and they are not the only example that can be used.

As used herein, the term "stem cell" refers to a cell capable of self-replication and pluripotency. Typically, stem cells can regenerate an injured tissue. Stem cells used herein may be, but are not limited to, embryonic stem (ES) cells or tissue stem cells (also called tissular stem cell, tissue-specific stem cell, or somatic stem cell), A stem cell may be an artificially produced cell as long as it can have the above-described abilities. Embryonic stem cells are pluripotent stem cells derived from early embryos. An embryonic stem cell was first established in 1981, and has been applied to production of knockout mice since 1989. In 1998, a human embryonic stem cell was established, which is currently becoming available for regenerative medicine. Tissue stem cells, unlike embryonic stem cells, are limited in the direction of differentiation. Tissue stem cells are present at specified positions in tissues and have an undifferentiated intracellular structure. Therefore, tissue stem cells have low level of pluripotency. Tissue stem cells have a higher nucleus/cytoplasm ratio with few intracellular organelles. Most tissue stem cells have pluripotency, a long cell cycle, and proliferative ability beyond the life of the individual. As used herein, stem cells may preferably be tissue stem cells such as mesenchymal stem cells and epithelial stem cells but depending on the circumstances, embryonic stem cells may also be employed. These tissue stem cells can be treated with dispase in order to be isolated from the tissue.

As used herein, the term "stem cells" is understood to refer to a tissue assemblage containing at least a certain amount of stem cells. Therefore, as used herein, stem cells that can be used include, but are not limited to, stem cells that are recovered from adipose tissue by treatment with collagenase (such as the stem cells used in the Examples).

Tissue stem cells are divided into categories based on sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, hepatic stem cells, and the like. Tissue stem cells in the bone marrow system include hematopoietic stem cells, mesenchymal stem cells, and the like. Tissue stem cells in the nervous system include neural stem cells, retinal stem cells, and the like.

As used herein, the term "epithelial stem cells" means stem cells derived from epithelial tissues. Epithelial tissues include, but are not limited to, cornea, oral mucosa, skin, conjunctiva, bladder, renal tubules, kidney, digestive tracts (esophagus, stomach, small intestine, duodenum, and large intestine), liver, pancreas, mammary gland, salivary gland, lacrimal gland, prostate gland, hair root, trachea, lung, and the like.

As used herein, the term "adipose stem cells" or "adipose precursor cells" means stem cells derived from adipose tissue. Some of the methods of isolating these stem cells are known and the methods described in, for example, "Protocols of Studies on Stem Cells and Clones", ed, by Nakatsuji, Yohdosha (2001) (in Japanese) may be used to perform the intended isolation. The information described in this and other references, in particular, those portions which are relevant to the present invention, may be incorporated herein by reference. Alternatively, adipose stem cells can be prepared by making use of the methods described in WO 00/53795; WO 03/022988; WO 01/62901; Zuk. P.A. et al., Tissue Engineering, Vol. 7, 211-228, 2001; Zuk, P.A. et al., Molecular Biology of the Cell, Vol., 13, 4279-4295, 2002 and the like, or modifications thereof. Specifically, the following protocol may be used to prepare adipose stem cells: (1) thoroughly wash a mass of aspirated fat with physiological saline using a separating funnel; (2) confirm that the aspirated fat has separated into the upper layer and the physiological saline into the lower layer, and discard the lower layer; repeat this step until the physiological saline becomes almost transparent to the naked eye; (3) add 0.075% collagenase/PBS in the same amount as the aspirated fat and incubate the mixture for 30 minutes at 37°C with thorough agitation; (4) add an equal volume of DMEM supplemented with 10% serum to the sample; (5) centrifuge the sample at 1200g for 10 minutes; (6) add 0.16 M NH₄Cl/PBS to the pellet to form a suspension and incubate it at room temperature for an appropriate period (e.g. 10-15 minutes); (7) filter the sample by suction through a mesh having 100 µm of pores; and (8) centrifuge the filtrate at 1200g for 5 minutes to recover adipose stem cells. Note that it is within the technical expertise of a skilled artisan to scale up or scale down the protocol depending upon the size of preparation. The present invention has revealed that the thus prepared adipose stem cells can be used as feeder cells. The adipose stem cells to be used as feeder cells may be prepared by such a step that the desired cells are obtained in a closed system when specified tissue stem cells are obtained from a tissue section recovered from a living tissue using a puncture needle.

As used herein, such adipose stem cells may be identified by various means that include, but are not limited to, cell marker and the properties of cytokines secreted from cells.

Exemplary cell markers include, but are not limited to, cell surface markers selected from the group consisting of CD4, CD13, CD34, CD36, CD49d, CD71, CD90, CD105, CD117 and CD151, or from the group consisting of CD105, CD73, CD29, CD44 and Sca-1. Alternatively, mesenchymal stem cells are said to have the surface antigen CD105(+), CD73(+), CD29(+), CD44(+), CD14(-), CD34(-) or CD45(-) and it will be understood that cells that exhibit at least one, preferably at least two, more preferably all, of these properties are preferred as cells that should be used in the present invention. In the present invention, lin(-)Scal(+) cells were isolated from bone marrow mononuclear cells by the magnetic beads method. When the isolated cells were cultured as described above, they grew to fibroblast-like cells in the group using adipose precursor cells. These cells were not fully studied but it is interesting to note that in the case of BL6 mouse, mesenchymal stem cells were reported to be lin(-)Scal(+)ckit(-) (see Peister et al., Blood. 2004 Mar 1;103(5):1662-8) (hematopoietic stem cells are lin(-)Scal(+)ckit(+)).

Cells conditioned by treating human subcutaneous adipose tissue with collagenase were cultured under conditions of 0.5 mM IBMX, 1 µM dexamethasone, 10 µM insulin and 200 µM indomethacin and they turned out positive in Oil red staining, so they were shown to be cells capable of induced differentiation into adipose cells (adipogenesis). This property was retained even after passages. The said cellular population was also capable of differentiation into neuronal cells (neurogenesis) by stimulation with 5 mM beta-mercaptoethanol and when they were cultured in EGM medium supplemented with EGF and VEGF on a collagen-coated culture dish, differentiation into endothelial cells (CD31 positive) was also confirmed (vasculogenesis). Previous reports have shown that 30% of the cells in this population have the characteristics of CD34 positive cells, namely hematopoietic stem cells, and they are considered to form a population having very high capability of differentiating into blood vessel constituting cells; therefore, a further study was made in the present invention focusing on vasculogenesis therapy.

Cytokines secreted by the adipose stem cells to be used in the present invention include, but are not limited to, VEGF, LIF, Lit ligand, MIP1 alpha (chemokine), IL-6, G-CSF, FGF and IGF. It has been found in the present invention that a specified group of cells that can be used as feeder cells express and secrete a particularly large amount of VEGF. Therefore, although not wishing to be bound by any theory, such cells as can secrete a large amount of VEGF can preferably be used as feeder cells in the system for regenerative therapy of the present invention. Such cells as secrete cytokines like VEGF may be separated from tissues as a primary cultured cells: alternatively, a nucleic acid molecule that contains a nucleic acid sequence coding for the cytokine of interest may be transferred into a cell by gene manipulation (e.g. transformation, transduction or transfection) to make the cytokine-secreting cell. It should be understood that such genetically modified cells can also be used in the present invention.

As used herein, the term "fibroblasts" means cells that supply the fiber component of a supportive tissue and comprise an important part of the fibrous connective tissue. When viewed in a tissue section, a fibroblast has a flat oblong appearance, often presenting irregular projections. The cytoplasm contains mitochondria, Golgi apparatus, centromere, small fat particles, etc. but it does not show any other specialized differentiation. The nucleus is oval in shape and often occurs in close contact with collagen fibers. It is said that fibroblasts isolated from the adipose tissue contain a lot of stem cells. In the present invention, it has been found that fibroblasts isolated from the adipose tissue, the sources of which are abundant within the body, are suitable as feeder cells and the present inventors have applied such fibroblasts in regenerative therapy. In the present invention, undifferentiated cells such as stem cells, precursor cells and fetal cells can also be feeder cells that can be used.

The cells to be used in the present invention may be xenogeneic or homogeneous, with the latter being preferred; they may be allogeneic or syngeneic, with the latter being more preferred and advantageous; however, it should be understood that xenogeneic cells can also be used.

As used herein, the term "somatic cell" refers to any cell other than a germ cell, such as an egg, a sperm, or the like, which does not directly transfer its DNA to the next generation. Typically, somatic cells have limited or no pluripotency. Somatic cells used herein may be naturally occurring or genetically modified.

As used herein, the term "differentiated cell" refers to a cell having a specialized function and morphology (e.g., muscle cells, neurons, etc.). Unlike stem cells, differentiated cells have no or little pluripotency. Examples of differentiated cells include epidermic cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, adipose cells, bone cells, cartilage cells, and the like. If they are to be used in the present invention, differentiated cells may be in the form of a population or tissue.

Therefore, as used herein, the term "differentiation" generally means the separation of a single system into two or more systems that differ in nature; if the term is to used for cells, tissues or organs, it means specialization of function and/or morphology. Differentiation is typically accompanied by decreased or lost pluripotency.

As used herein, the "differentiating factor", also known as "differentiation promoting factor", may be any factor as long as it is a factor (e.g. chemical substance or temperature) that is known to promote the differentiation to a differentiated cell. A variety of environmental factors may be mentioned as such factors and they include, but are not limited to, temperature, humidity, pH, salt concentration, nutrition, metal, gas, organic solvent, pressure, chemical substance (e.g. steroid or antibiotic) and the like, as well as any combinations thereof. Typical differentiating factors include, but are not limited to, cytophysiologically active substances. Typical examples of these factors include, but are not limited to, DNA demethylating agents (e.g. 5-azacytidine), histone deacetylating agents (e.g. trichostatin), nuclear receptor ligands (e.g. retinoic acid (ATRA), vitamin D₃ and T3), cell growth factors (e.g. activin, IGF-1, FGF, PDGF, TGF-beta, and BMP2/4), cytokines (e.g. LIF. IL-2, and IL-6), hexamethylene bisacetamide, dimethylacetamide, dibutyl cAMP, dimethyl sulfoxide, iododeoxyuridine, hydroxylurea, cytosine arabinoside, mitomycin C, sodium butyrate, aphidicolin, fluorodeoxyuridine, polybrene, and selenium.

Media for culturing animal cells that may be used in the present invention include cysteine protease inhibitors. Cysteine protease inhibitors are proteins that specifically inhibit cysteine protease and are deeply involved in the homeostasis maintenance mechanism and the immune protection mechanism; although the mechanism of their action is not known, it has been found that use of media containing cysteine protease inhibitors contributes to enhancing the ability of animal cells to form colonies during culture. Specific examples of cysteine protease inhibitors include cystatins and their superfamily bromelain inhibitors but they are not limited to any particular types as long as they can function as cysteine protease inhibitors. In the present invention, each cysteine protease inhibitors may be used alone or in combination with other one or more inhibitors.

It is also preferred that the media for culturing animal cells that may be used in the present invention contain cystatins and their superfamily. The cystatins and their superfamily may or may not function as the above-described cysteine protease inhibitors; specific examples include phytocystatins such as oryzacystatin, cystatin, cystatin A, cystatin B, cystatin C and monellin but the cystatins and their superfamily are not limited to any particular types as long as they belong to the cystatin superfamily. In the present invention, each cystatins and their superfamily may be used alone or in combination with one or more substances.

In the present invention, at least one substance selected from the group consisting of cysteine protease inhibitors, cystatin C and substances that belong to its superfamily is added to a medium for culturing animal cells; the total amount of such substances is desirably in the range of concentration from 0.5 ng/ml to 10 ng/ml, preferably from 0.6 ng/ml to 9 ng/ml, more preferably from 0.8 ng/ml to 8 ng/ml, on the basis of the volume of the medium. If their concentration is smaller than 0.5 ng/ml, they are insufficient to maintain the activity of animal stem cells. At concentrations higher than 10 ng/ml, the activity is inhibited rather than enhanced.

Various growth factors may further be added in the present invention. Specific examples include, but are not limited to, EGF, FGF and HGF. The concentration at which they are added is desirably at least 2 ng/ml, preferably at least 4 ng/ml, and more preferably at least 10 ng/ml. Concentrations smaller than 2 ng/ml are insufficient since no effect of addition is recognized.

The present invention employs the above-described media to improve the ability of animal cells to form colonies during culture. The timing on which such media are to be employed is not limited in any particular way and they may be employed throughout the period of culture from beginning to end; alternatively, they may be substituted in part of the culture period for conventionally used media. However, in order to maintain the activity of the cells being cultured, the media of interest are advantageously used throughout the period of culture.

Examples of the base material for culture include not only materials typically used in cell culture (e.g., glass, modified glass, compounds such as polystyrene and polymethyl methacrylate) but also all materials that can be generally shaped (e.g., macromolecule compounds other than those described above, ceramics, etc.). One may also use base materials for culture that are coated with collagen, fibronectin, laminin, poly-L-lysine, matrigel and the like for various purposes such as enhancing the adhesion to the base material of the cells being cultured.

Cells that are preferably used in the present invention include epidermal cells such as those of the skin, epithelial cells such as those of the cornea, liver, digestive organs, mammary gland, prostate gland, hair root, trachea and oral mucosa, as well as stem cells thereof; the kinds of cells that can be used are by no means limited. Other conditions of culture may be in compliance with the usual method and are not limited in any particular way. For example, the medium to be used may be one supplemented with serum such as known fetal calf serum (FCS) or it may be a serum-free medium containing none of such sera. Preferably, considering the risk of Bovine Spongiform Encephalopathy (BSE), it can be advantageous to use sera other than bovine-derived sera.

Employing the media that can be used in the present invention to culture animal cells, one can maintain the activity of cells and stem cells. In the case of cells that will be layered by using such media, they can be layered without using the already described xenogeneic cells from an animal of a different species. This is a feature that would prove very effective in providing a technology of regenerative medicine that involves tissue regeneration and cell differentiation.

As used herein, the term "cytophysiologically active substance" or "physiologically active substance" refers to a substance capable of acting on a cell or tissue. Examples of such action include, but are not limited to, the control or change of the cell or tissue of interest. Physiologically active substances include cytokines and growth factors. The physiologically active substance may be naturally occurring or synthesized. Preferably, the physiologically active substance may be one that is produced by a cell or one that has a function similar thereto; if desired, it may have a modified action. As used herein, the physiologically active substance may be in the form of a protein or in other forms but at the time when they act actually, cytokines usually means proteinaceous forms.

As used herein, the term "cytokine" is defined to have the broadest sense in the art and refers to a physiologically active substance which is produced from a cell and acts on the same or different cells. Cytokines are generally proteins or polypeptides having a controlling function of an immune response, a regulating function of the endocrine system, a regulating function of the nervous system, an anti-tumor function, an anti-virus function, a regulating function of cell growth, a regulating function of cell differentiation, or the like. As used herein, cytokines can be in the form of a protein or in other forms but at the time when they act actually, cytokines usually means proteinaceous forms.

As used herein, the terms "growth factor" and "cell growth factor" are used interchangeably and each refers to a substance which promotes or controls cell growth. Growth factors are also called "proliferation factors" or "development factors". Growth factors may be added to cell or tissue culture medium, replacing the action of serum macromolecules. It has been revealed that a number of growth factors have a function of controlling differentiation in addition to a function of promoting cell growth.

Examples of cytokines representatively include, but are not limited to, interleukins, chemokines, hematopoietic factors such as colony stimulating factors, a tumor necrosis factor, and interferons. Growth factors typically include those which have proliferative activity, as exemplified by a platelet-derived growth factor (PDGF), an epidermal growth factor (EGF), a fibroblast growth factor (FGF), a hepatocyte growth factor (HGF), a vascular endothelial growth factor (VEGF) and the like.

Physiologically active substances such as cytokines and growth factors typically have redundancy in function. Accordingly, a cytokine or growth factor can be used in the present invention as long as the cytokine or growth factor has the activity of a physiologically active substance for use in the present invention, even though the cytokine or growth factor is known to those of skill in the art by any other names or functions (e.g. cell adhesion activity or cell-substrate adhesion activity). Cytokines or growth factors can be used in preferred embodiments of the present invention as long as they have preferred activities (e.g. activity capable of inducing desired differentiation) as described herein.

Specific differentiating factors include the following, which may be used either alone or in combination with each other.
A) cornea: epidermal growth factor (EGF);
B) skin (keratinocyte): TGF-beta, FGF-7 (KGF: keratinocyte growth factor), EGF
C) vascular endothelium: VEGF, FGF, angiopoietin
D) kidney: LIF, BMP, FGF, GDNF
E) heart: HGF, LIF, VEGF
F) liver: HGF, TGF-beta, IL-6, EGF, VEGF
G) umbilical endothelium: VEGF
H) intestinal epithelium: EGF, IGF-I, HGF, KGF, TGF-beta, IL-11
I) nerve: nerve growth factor (NGF), BDNF (brain-derived neurotrophic factor), GDNF (glial cell-line derived neurotrophic factor), neurotrophin, IL-6, TGF-beta, TNF
J) glial cell: TGF-beta, TNF-alpha, EGF, LIF, IL-6
K) peripheral neuron: bFGF, LIF, TGF-beta, IL-6, VEGF
L) lung (pulmonary alveolar epithelium): TGF-beta, IL-13, IL-lbeta, KGF, HGF
M) placenta: growth hormone (GH), IGF, prolactin, LIF, IL-1, activin A. EGF
N) pancreatic epithelium: growth hormone, prolactin
O) pancreatic Langerhans cell (islet cell): TGF-beta, IGF, PDGF, EGF, TGF-beta, TRH (thyrotropin-releasing hormone)
P) epithelium of the synovial membrane of a joint: FGF, TGF-beta
Q) osteoblast: BMP, FGF
R) chondroblast: FGF, TGF-beta, BMP, TNF-alpha,
S) retinal cell: FGF, CNTF (ciliary neurotrophic factor)
T) adipose cell: insulin, IGF, LIF
U) muscle cell: LIF, TNF-alpha, FGF.

As used herein, the term "maintaining" in relation to cells, tissues or organs, means substantially retaining their function and/or morphology. For example, maintenance of the cornea means that a subject has the cornea without substantially damaging the function and/or morphology the subject should normally have; cornea's functions include, but are not limited to, the maintenance of visual acuity and its forms include, but are not limited to, the retention of the appearance.

As used herein, the term "regeneration" refers to a phenomenon in which when an individual organism loses a portion of tissue or organ, the remaining tissue grows and recovers. The extent or manner of regeneration differs from one animal species to another or from one tissue from another in the same individual. Most human tissues have limited regeneration capability, and therefore, complete regeneration is not expected if a large portion of tissue is lost. In the case of severe damage, a tissue may grow which has strong proliferation capability different from that of lost tissue, resulting in incomplete regeneration where the damaged tissue is incompletely regenerated and the function of the tissue cannot be recovered. In this case, a structure made of a bioabsorbable material is used to prevent a tissue having strong proliferation capability from infiltrating the defective portion of the tissue so as to secure space for proliferation of the damaged tissue. Further, by supplementing with a cell growth factor, the regeneration capability of the damaged tissue is enhanced. Such a regeneration technique is applied to cartilages, bones, and peripheral nerves, for example. It has been so far believed that neuronal cells and cardiac muscles have no or poor regeneration capability. Recently, it was reported that there are tissue stem cells (somatic stem cells) which have both the capability of differentiating into these tissues and self-proliferation capability and this had led to an increased expectation of regenerative medicine using such tissue stem cells.

A stem cell is categorized into the ectoderm-derived, mesoderm-derived, or endoderm-derived stem cell based on the origin of the stem cell. Stem cells of ectodermal origin are mostly present in the brain, including neural stem cells and their differentiated cells. Stem cells of mesodermal origin are mostly present in bone marrow, including blood vessel stem cells or their differentiated cells, hematopoietic stem cells or their differentiated cells, and mesenchymal stem cells or their differentiated cells, and the like. Stem cells of endoderm origin are mostly present in organs, including hepatic stem cells or their differentiated cells, pancreatic stem cells or their differentiated cells, and the like. As used herein, somatic cells may be derived from any germ layers. Preferably, somatic cells derived from mesenchyme may be employed.

As used herein, the terms "feeder layer" and "feeder cell" can be used interchangeably and refer to that support cell layer on a culture substrate which is formed of a certain cell species such that it enables the growth of and/or differentiated phenotypic expression of another cell species that cannot be cultured and maintained independently. It is said that tissue cells comprise many cell species that cannot grow, still less achieve differentiated phenotypic expression, and examples of such cells include, but are not limited to, certain kinds of stem cells (in particular, epithelial stem cells, embryonic stem cells, and hematopoietic stem cells), as well as cornea and epidermis. These cell species in general are highly auxotrophic and are said to require specific growth factors or differentiation inducing factors. It is said for these cell species that if a layer of specified cells that are formed by support cells for that cell species *in vivo* or similar cells thereto is utilized as a culture substrate, the factors and/or nutrient sources that are required by the cell species to be cultured are supplied, causing those cells species to grow and differentiate. The cell species to be used as feeder cells is selected depending on the cell species to be cultured and cell growth is often suppressed by administering antibiotics (e.g. mitomycin C) or UV irradiation. It is said that culture of germ cells, early embryo cells, hematopoietic stem cells and the like has become possible, largely on account of the effective use of feeder layers. As feeder cells, not only fat derived stem cells but also embryonic stem cells, bone marrow stem cells and the like can be used and in that case, it is preferred to perform selective culture of the feeder under the most basic culture conditions using DMEM + 10% FBS so that it is differentiated into fibroblasts for further use.

As used herein, the term "primary cultured cells" means those cells which were derived from a cell, tissue or organ as separated from the body and have subsequently been cultured to a stage just before the first passage.

The cells of the present invention can be cultured using any desired culture media as long as they can be maintained or can differentiate into desirable differentiated cells. Examples of such culture media include, but are not limited to, DMEM, P199. MEM, HBSS, Ham's F12, BME, RPMI 1640, MCDB 104, MCDB 153 (KGM), and mixtures thereof. These culture solutions may contain adrenocorticosteroid such as dexamethasone, insulin, glucose, indomethacin, isobutyl-methylxanthine (IBMX), ascorbate-2-phosphate, ascorbic acid and derivatives thereof, glycerophosphate, estrogen and derivatives thereof, progesterone and derivatives thereof, androgen and derivatives thereof, growth factors such as aFGF, bFGF, EGF, IGF, TGFbeta, ECGF, BMP and PDGF, pituitary gland extract, pineal body gland, retinoic acid, vitamin D, thyroid hormone, fetal calf serum, horse serum, human serum, heparin, sodium hydrogencarbonate, HEPES, albumin, transferrin, selenic acid (e.g. sodium selenite), linolenic acid, 3-isobutyl-1-methylxanthine, demethylating agents such as 5-azacytidine, histone deacetylating agents such as trichostatin, activin, cytokines such as LIF, IL-2, and IL-6, hexamethylene bisacetamide (HMBA), dimethylacetamide (DMA), dibutyl cAMP (dbcAMP), dimethyl sulfoxide (DMSO), iododeoxyuridine (IdU), hydroxylurea (HU), cytosine arabinoside (AraC), mitomycin C (MMC), sodium butyrate (NaBu), polybrene, selenium, cholera toxin and the like, each of which may be used either alone or in combination with each other.

In the present invention, a temperature culture dish is advantageously used in culture; although this is not the sole case of the present invention, it is preferred since it allows for easy separation of cells. The temperature culture dish is typically one that is coated with a temperature-responsive polymer. The temperature culture dish may be what is commercially available from CellSeed Inc. (Tokyo, Japan).

As used herein, the term "temperature-responsive polymer " refers to a polymer which changes its shape and/or property in response to change of temperature. Examples of temperature-responsive polymers include, but are not limited to, poly(N-isopropylacrylamide), poly(N-isopropylacrylamide-acrylic acid) copolymer, poly(N-isopropylacrylamide-methyl methacrylate) copolymer, poly(N-isopropylacrylamide-sodium acrylate) copolymer, poly(N-isopropylacrylamide-vinylferrocene) copolymer, gamma ray-exposed poly(vinyl methyl ether) and a gel obtained by cross-linking the above-described polymers with a cross-linking agent, and the like. Preferable examples of temperature-responsive polymers include, but are not limited to, poly(N-isopropylacrylamide), poly(N-isopropylacrylamide-methyl methacrylate) copolymer, poly(N-isopropylacrylamide-sodium acrylate) copolymer, and a gel obtained by cross-linking the above-described polymers with a cross-linking agent, and the like. For example, a temperature-responsive polymer used herein has an upper or lower critical solution temperature in water of from 0°C to 80°C. The present invention is not limited to this. The term "critical solution temperature" of a polymer refers to a temperature threshold which changes a shape and/or property of the polymer. Preferably, poly(N-isopropylacrylamide) may be herein used.

For example, gamma-ray-exposed polyvinyl methyl ether forms a hydrate at room temperature in aqueous solution which in turn swells. It is known that as the temperature is increased, the substance is dehydrated, so that the solution is contracted and turned into a heat-sensitive polymer gel. The PVME gel, which is heterogeneous and transparent like a jelly, is turned cloudy (i.e., its transparency is changed) if it is heated. If the gel is provided with a porous structure or formed into minuscule shapes such as fibers or particles, the gel can extend or contract at high speed. It is believed that a porous and fiber-like PVME gel can extend or contract within less than one second (see http://www.aist.go.jp/NIMC/overview/v27-j.htmL, Japanese Laid-Open Publication No. 2001-213992, and Japanese Laid-Open Publication No. 2001-131249). N-isopropylacrylamide gel (i.e., poly(N-isopropylacrylamide)) is also known as a temperature-responsive gel. If poly(N-isopropylacrylamide) is copolymerized with a hydrophobic monomer, the temperature which allows it to change its shape and/or property can be lowered. If poly(N-isopropylacrylamide) is copolymerized with a hydrophilic monomer, the temperature which allows it to change its shape and/or property can be raised. By utilizing this character, it is possible to prepare a filler responsive to a desired stimulus. Such a technique can be applied to other temperature-responsive polymers.

As used herein, the term "established" in relation to cells refers to a state of a cell in which a particular property (e.g. pluripotency) of the cell is maintained and the cell undergoes stable proliferation under culture conditions. Therefore, established stem cells maintain pluripotency. Established differentiated cells have a specific determined function. Established differentiated cells are often found cancerous but this is not the sole case.

As used herein, the term "within the living body" or "*in vivo*" refers to the inner part of the living body. In a specific context, "within the living body refers to a position at which a subject tissue or organ should be located.

As used herein, the term *"in vitro"* indicates that a part of the living body is excised or released outside the living body for various purposes of research (e.g., in a test tube). The term "*in vitro*" is in contrast to the term "*in vivo.*"

As used herein, the term *"ex vivo"* refers to a series of operations where target cells into which a gene will be introduced are extracted from a subject; a therapeutic gene is introduced *in vitro* into the cells; and the cells are returned into the same subject.

As used herein, the term "self" or "autologous" in relation to a certain individual refers to either an individual derived from that individual or a part thereof (a cell, a tissue, an organ, etc.). As used herein, the term "autologous" may encompass a graft from a genetically identical individual (e.g. an identical twin) in a broad sense.

As used herein, the expression "the same species" or "allogeneic" refers to an individual or a part thereof (a cell, a tissue, an organ, etc.) which is implanted from one which is of the same species but genetically different. Since an allogeneic implant is genetically different from an individual (recipient) to which it is implanted, it may elicit an immune reaction. Cells which are allogeneic include, but are not limited to, a cell derived from a parent.

As used herein, the expression "a different species" refers to what is implanted from an individual of a different species. Therefore, if a human is a recipient, a porcine-derived graft is called a xenograft.

As used herein, "recipient" (acceptor) refers to an individual who receives a cell and the like which are to be implanted and is also called "a host". In contrast, an individual providing the cell and the like to be implanted is called "a donor" (provider). The recipient and the donor may be the same or different individual.

Cells that are used in the present invention may be derived from a syngeneic origin (self or autologous origin), an allogeneic origin (non-self or heterologous origin), or a xenogeneic origin. In view of rejection reactions, cells of a self origin are preferable. If rejection reactions do not raise problems, autologous cells may be employed.

As used herein, the term "grafting" or "implantation" means that the cell, composition, pharmaceutical preparation and the like of the present invention are introduced separately or in combination with other therapeutic agents. The present invention permits use of the following methods of introduction into therapy sites (e.g., bone, etc.), the following modes and the following amounts: examples of the introduction methods include, but are not limited to, a method in which the pharmaceutical preparation of the present invention is introduced by directly attaching it to the impaired site, and suturing it after attachment, and the like. The adipose stem cells of the present invention in combination with differentiated cells may be administered either concomitantly (e.g., as an admixture), separately but simultaneously or concurrently; or sequentially. This includes presentations in which the combined agents are administered together as a therapeutic mixture, and also procedures in which the combined agents are administered separately but simultaneously (e.g., a differentiation promoting factor). The present invention further includes a "combination" administration or implantation method in which the first introduction of one of the cells, pharmaceutical preparations, compounds or agents into the body is conducted separately from the second introduction of one of the cells, pharmaceutical preparations, compounds or agents.

As used herein, the term "grafting" or "implantation" means that the cell, composition, pharmaceutical preparation and the like of the present invention are administered singly or in combination with other therapeutic agents. The present invention permits use of the following methods of introduction into therapy sites (e.g., impaired heart, etc.) in the following forms and in the following amounts: examples of the introduction methods include, but are not limited to, direct attachment to the impaired site of the pharmaceutical preparation of the present invention, suture after attachment, insertion, and the like. The adipose stem cells of the present invention in combination with differentiated cells may be administered either concomitantly (e.g., as an admixture). separately but simultaneously or concurrently; or sequentially. This includes presentations in which the combined agents are administered together as a therapeutic mixture, and also procedures in which the combined agents are administered separately but simultaneously (e.g., a differentiation promoting factor). "Combination" administration or grafting or implantation further includes the separate administration of one of the cells, pharmaceutical preparations, compounds or agents given first, followed by the second.

The disease, disorder or condition that can be dealt with by the method of the present invention encompasses any disease, disorder or condition in which the regeneration of an organ or tissue is desired. The present invention is particularly advantageous if it is to deal with a disease, disorder or condition that are related to an organ, tissue or cell that cannot be regenerated without feeder cells.

In one embodiment, the disease or disorder that can be dealt with by the present invention can be an ophthalmic disease or disorder. Examples of such disease or disorder include, but are not limited to, thermal corrosion, alkali corrosion, acid corrosion, drug toxicity, Stevens-Johnson syndrome, ocular pemphigoid, (recurrent) pterygium, persistent corneal epithelium defect, corneal perforation, circumcorneal ulcer, corneal ulcer, epithelial detachment after surgical operation with an excimer laser, radiation keratopathy, aniridia, post-trachoma corneal opacity, Salzmann corneal dystrophy, corneal erosion, adhesion of eyeball, disease characterized by unaccountable loss of corneal epithelial stem cells, limbus tumor, graft-versus-host disease (GVHD), and the like.

In one embodiment, the disease or disorder that can be dealt with by the present invention can be an epithelium-associated disease or disorder. Examples of such disease or disorder include, but are not limited to, skin disease, intestinal disease, airway disease, oral disease, bladder disease, gonaduct disease, corneal disease, and the like.

In one embodiment, the disease or disorder that can be dealt with by the present invention can involve the circulatory system (e.g. blood cells). Examples of such disease or disorder include, but are not limited to, anemia (e.g. aplastic anemia (in particular, grave aplastic anemia), renal anemia, carcinomatous anemia, secondary anemia, and refractory anemia), cancer or tumor (e.g. leukemia) and dyshemopoiesis after their treatment by chemotherapy, thrombocytopenia, acute myelocytic leukemia (in particular, remissions after the first remission (high-risk group) and the second remission), acute lymphocytic leukemia (in particular, remissions after the first remission and the second remission), chronic myelocytic leukemia (in particular, chronic and transitional periods), malignant lymphoma (in particular, remissions after the first remission (high-risk group) and the second remission), multiple myeloma (in particular, early period after manifestation); heart failure, angina pectoris. myocardial infarction, arrhythmia, valvular disorder, myocardial or pericardium disease, congenital heart disease (e.g. atrial septal defect, ventricular septal defect, arterial canal patency, and tetralogy of Fallot), arterial disease (e.g. arteriosclerosis and aneurysm), venous disease (e.g. varix), lymphoduct disease (e.g. lymphatic edema), and the like.

In another embodiment, the disease or disorder that can be dealt with by the present invention can involve the nervous system. Examples of such disease or disorder include, but are not limited to, dementia, stroke and its sequelae, brain tumor, damaged spinal cord, and the like.

In another embodiment, the disease or disorder that can be dealt with by the present invention can involve the immune system. Examples of such disease or disorder include, but are not limited to, T cell defect, leukemia, and the like.

In another embodiment, the disease or disorder that can be dealt with by the present invention can involve the locomotorial/skeletal system. Examples of such disease or disorder include, but are not limited to, bone fracture, osteoporosis, joint displacement, subluxation, distortion, damaged ligament, osteoarthritis, osteosarcoma, Ewing sarcoma, dysosteogenesis, myodystrophy, osseocartilaginous dysplasia, and the like.

In another embodiment, the disease or disorder that can be dealt with by the present invention can involve the skin system. Examples of such disease or disorder include, but are not limited to, atrichia, melanoma, malignant dermal lymphoma, angiosarcoma, histiocytosis, hydroa, pustulosis, dermatitis, eczema, and the like.

In another embodiment, the disease or disorder that can be dealt with by the present invention can involve the endocrine system. Examples of such disease or disorder include, but are not limited to, hypothalamohypophysial disease, thyroid disease, parathyroid disease, adrenooortical medullary disease, abnormal glucose metabolism, abnormal lipid metabolism, abnormal protein metabolism, abnormal nucleic acid metabolism, inborn errors of metabolism (phenylketonuria, galactosemia, homocystinuria, and maple syrup urine disease), analbuminemia, lack of ascorbic acid synthesizing ability, hyperbilirubinemia, hyperbilirubinuria, kallikrein defect, mast cell defect, diabetes insipidus, abnormal vasopressin secretion, dwarfism, Wolman disease (acid lipase deficiency), type VI mucopolysaccharidosis, and the like.

In another embodiment, the disease or disorder that can be dealt with by the present invention can involve the respiratory system. Examples of such disease or disorder include, but are not limited to, pulmonary disease (e.g. pneumonia and lung cancer), bronchial disease, and the like.

In another embodiment, the disease or disorder that can be dealt with by the present invention can involve the digestive system. Examples of such disease or disorder include, but are not limited to, esophageal disease (e.g. cancer of the esophagus), stomach/duodenum disease (e.g. stomach cancer and duodenum cancer), small or large intestine disease (e.g. polyp in the large intestine, colon cancer, and rectum cancer), bile duct disease, liver disease (e.g. cirrhosis, hepatitis (e.g. type A, B, C, D or E), fulminant hepatitis, chronic hepatitis, primary liver cancer, alcoholic hepatopathy, drug-induced hepatopathy), pancreatic disease (acute pancreatitis, chronic pancreatitis, pancreatic cancer, cystic pancreatic disease), peritoneum/abdomen/diaphragm disease (e.g. hernia), Hirschsprung's disease, and the like.

In another embodiment, the disease or disorder that can be dealt with by the present invention can involve the urinary system. Examples of such disease or disorder include, but are not limited to, renal disease (e.g. renal failure, primary glomerular disease, renovascular disorder, abnormal tubule function, stromal renal disease, renal disorder due to systemic disease, and kidney cancer), bladder disease (e.g. cystitis and bladder cancer), and the like.

In another embodiment, the disease or disorder that can be dealt with by the present invention can involve the genital system. Examples of such disease or disorder include, but are not limited to, male genital disease (e.g. male infertility, prostatomegaly, prostate cancer, and testis cancer), female genital disease (e.g. female infertility, impaired ovary function, myometrium, adenomyosis uteri, uterus cancer, endometriosis, ovary cancer, and villous disease), and the like.

Animals targeted by the present invention include any animals (e.g., Myxiniformes, Petronyzoniformes, Chondrichthyes, Osteichthyes, amphibian, reptilian, avian, mammalian, etc.) as long as it has epithelial cells or fat cells. Preferably, such animals can be mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). Illustrative examples of a subject include, but are not limited to, animals, such as cattle, pigs, horses, chickens, cats, dogs, and the like. More preferably, primates (e.g., chimpanzee, Japanese monkey, human, etc.) are used. Most preferably, a human is used. What should be particularly noted about the beneficiary effects of the present invention is that the criteria of the competent authority can be met fairly easily and that it has become easy to use human-derived cells.

When the present invention is used as a pharmaceutical agent, it may further comprise a pharmaceutically acceptable carrier or the like. A pharmaceutically acceptable carrier contained in a medicament of the present invention includes any material known in the art.

Examples of such a pharmaceutically acceptable carrier include, but are not limited to, antioxidants, preservatives, colorants, flavoring agents, diluents, emulsifiers, suspending agents, solvents, fillers, bulking agents, buffers, delivery vehicles, excipients and/or pharmaceutical adjuvants, and the like. Typically, the medicament of the present invention is administered in the form of a composition comprising the cells of the present invention together with at least one physiologically acceptable carrier, excipient or diluent. For example, suitable vehicles can be water for injection, physiological solution or artificial cerebrospinal solution and these can be supplemented with other substances that are generally used with compositions for parenteral delivery.

The acceptable carrier, excipient or stabilizer that can be used in the present invention is nontoxic to the recipient and is preferably inert at the dose and concentration used; examples include, but are not limited to, phosphates, citrates and other organic acids; ascorbic acid and alpha-tocopherol; low-molecular weight polypeptides; proteins (e.g. serum albumin, gelatin or immunoglobulin); hydrophilic polymers (e.g. polyvinyl pyrrolidone): amino acids (glycine, glutamine, asparagine, arginine, or lysine); monosaccharide, disaccharide and other carbohydrates (including glucose, mannose, or dextrin); chelating agents (e.g. EDTA); sugar alcohols (e.g. mannitol or sorbitol); salt forming counter ions (e.g. sodium); and/or nonionic surfactants (e.g. Tween, Pluronic or polyethylene glycol (PEG)), and the like.

Suitable exemplary carriers include neutral buffered physiological saline or physiological saline mixed with serum albumin. Preferably, the resulting product is formulated as a freeze-dried agent using a suitable excipient (e.g. sucrose). Other standard carriers, diluents and excipients may be contained as desired. Other exemplary compositions comprise a Tris buffer of pH 7.0-5.5 or an acetate buffer of pH 4.0-5.5. which may further comprise sorbitol or any suitable substitutes for it.

On the following pages, a general method of preparing the pharmaceutical composition of the present invention is descried. Note that veterinary drug compositions, quasi-drugs, marine drug compositions, food compositions, cosmetic compositions and the like can also be produced by known methods of preparation.

The cells of the present invention, tissue grafts into which cells have differentiated, and the like may be mixed with pharmaceutically acceptable carriers to form injections, suspensions, solutions and other liquid preparations that may be administered perorally or parenterally; considering the treatment to be performed, the preparations are preferably administered parenterally. Pharmaceutically acceptable carriers include solvents, solvent promoters, suspending agents, isotonization agents, buffers, soothing agents and the like in liquid preparations. Depending on the need, pharmaceutical additives such as antiseptics, antioxidants, coloring agents, sweeteners and the like may be used. Substances other than the cells of the present invention can also be incorporated in the composition of the present invention, Routes for parenteral administration include, but are not limited to, intravenous, intramuscular, subcutaneous, intracutaneous, transmucosal, Intrarectal, intravaginal administration, as well as local administration to the diseased site, topical administration to the skin, and the like. When administered systemically, the medicament used in the present invention can be in the form of a pharmaceutically acceptable aqueous solution that does not contain any heat generating substance. Skilled artisans can easily prepare such pharmaceutically acceptable compositions by considering pH, isotonicity, stability, and the like.

Preferred examples of solvents in liquid preparations include water for injection, alcohol, propylene glycol, macrogol, sesame oil, corn oil, and the like.

Preferred examples of solvent promoters in liquid preparations include, but are not limited to, polyethylene glycol, propylene glycol, D-mannitol, benzyl benzoate, ethanol, trisaminomethane, cholesterol, triethanolamine, sodium carbonate, sodium citrate, and the like.

Preferred examples of suspending agents in liquid preparations include, but are not limited to, surfactants such as stearyl triethanolamine, sodium lauryl sulfate, lauryl aminopropionic acid, lecithin, benzalkonium chloride, benzethonium chloride, glycerol monostearate, and the like, and hydrophilic polymers such as polyvinyl alcohol, polyvinyl pyrrolidone, carboxymethyl cellulose sodium, methyl cellulose, hydroxymethyl cellulose, hydroxyethyl cellulose, hydroxypropyl cellulose, and the like.

Preferred examples of isotonization agents in liquid preparations include, but are not limited to, sodium chloride, glycerin, D-mannitol, and the like.

Preferred examples of buffers in liquid preparations include, but are not limited to, buffers such as phosphates, acetates, carbonates, citrates, and the like.

Preferred examples of soothing agents in liquid preparations include, but are not limited to, benzyl alcohol, benzalkonium chloride, procaine hydrochloride, and the like.

Preferred examples of antiseptics in liquid preparations include, but are not limited to, paraoxybenzoic acid esters, chlorobutanol, benzyl alcohol, 2-phenylethyl alcohol, dehydroacetic acid, sorbic acid, and the like.

Preferred examples of antioxidants in liquid preparations include, but are not limited to, sulfites, ascorbic acid, alpha-tocopherol, cysteine, and the like.

When prepared as an injection, the solution and suspending agent are preferably sterilized and rendered isotonic to the blood. Usually, these are made germ-free by filtration using a bacteria-retaining filter or the like, incorporating a sterilizer, or irradiation. After these treatments, freeze-drying or other suitable method may be applied to form a solid preparation, to which germ-free water or a germ-free diluent for injection (e.g. lidocaine hydrochloride in aqueous solution, physiological saline, glucose in aqueous solution, ethanol, or mixed solutions thereof) may be added just before use.

Depending on the need, the medicament of the present invention may contain coloring agents, preservatives, scents, flavoring agents, odorizers, sweeteners, and other drugs.

The amount of the composition to be used in the treatment method of the present invention can be easily determined by those skilled in the art with reference to the purpose of use, a target disease (type, severity, and the like), the patient's age, weight, sex, and case history, the form or type of the cell, and the like. The frequency of the treatment method of the present invention to be applied to a subject (or patient) can also be determined easily by those skilled in the art with reference to the purpose of use, target disease (type, severity, and the like), the patient's age, weight, sex, and case history, the progression of the therapy, and the like. Examples of the frequency include once per day to once in several months (e.g., once per week to once per month). Preferably, administration is performed once per week to once per month with reference to the progression. The dose to be administered can be established by estimating the amount that is required by the site to be treated.

As used herein, the "instructions" describe a method of administering a medicament, a method for diagnosis, or the like of the present invention for persons who administer, or are administered, the medicament or the like or persons who diagnose or are diagnosed (e.g., physicians, patients, and the like). The instructions describe a statement indicating an appropriate method for administering a diagnostic, a medicament, or the like of the present invention. The instructions are prepared in accordance with a format defined by an authority of a country in which the present invention is practiced (e.g., Health, Labor and Welfare Ministry in Japan, Food and Drug Administration (FDA) in the U.S. and the like), explicitly describing that the instructions are approved by the authority. The instructions are so-called package insert and are typically provided in paper media. The instructions are not so limited and may be provided in the form of electronic media (e.g., web sites, electronic mails, and the like provided on the Internet).

Judgment as to whether the medical treatment by the method of the present invention has ended can be supported by the result of a standard clinical test using a commercially available assay or apparatus or by the disappearance of clinical symptoms characteristic of a disease associated with deficiency of differentiated cells (e.g. ophthalmic disease, bone disease, heart disease or nerve disease). Medical treatment can be resumed if the disease associated with deficiency of differentiated cells (e.g. ophthalmic disease, bone disease, heart disease or nerve disease) recurs.

The present invention also provides a pharmaceutical pack or kit comprising at least one vessel filled with at least one component of the medicament of the present invention. A notice in the format specified by a governmental organization that regulates the manufacture, use or sales of pharmaceuticals or biological products can be attached as an optional appendage to the vessel and the notice represents the governmental organization's approval of manufacture, use or sales about administration to humans.

### (Description of the Preferred Embodiments)

Hereinafter, preferred embodiments of the present invention will be described. The following embodiments are provided for a better understanding of the present invention and the scope of the present invention should not be limited to the following description. It will be clearly appreciated by those skilled in the art that variations and modifications can be made without departing from the scope of the present invention with reference to the specification.

### (Feeder cells)

In one aspect, the present invention provides an adipose tissue derived for use as a feeder cell. While various cells can be isolated from adipose tissue, it is particularly preferred in the present invention that adipose tissue contains tissue stem cells or fibroblasts. Stem cells can be identified by confirming at least one indicator selected from the group consisting of CD105(+), CD73(+), CD29(+), CD44(+), CD14(-), CD34(-) and CD45(-). Alternatively, such cells are called "adipose stem cells" or "adipose progenitor cells". Such cells can be prepared by the following exemplary protocol: (1) thoroughly wash a mass of aspirated fat with physiological saline using a separating funnel; (2) confirm that the aspirated fat has separated into the upper layer and the physiological saline into the lower layer, and discard the lower layer; repeat this step until the physiological saline becomes almost transparent to the naked eye: (3) add 0.075% collagenase/PBS in the same amount as the aspirated fat and incubate the mixture for 30 minutes at 37°C with thorough agitation; (4) add an equal volume of 10% serum supplemented DMEM to the sample; (5) centrifuge the sample at 1200g for 10 minutes; (6) add 0.16 M NH₄Cl/PBS to the pellet to form a suspension and incubate it at room temperature for an appropriate period (e.g. 10-15 minutes); (7) filter the sample by suction through a screen having openings of 100 µm; and (8) centrifuge the filtrate at 1200g for 5 minutes.

Although not wishing to be bound by any theory, the feeder cells of the present invention have been observed secreting cytokines such as VEGF efficiently. Therefore, in a preferred embodiment, the feeder cells of the present invention have the nature of secreting VEGF or other cytokines and more preferably a cell population that contains cells that secrete VEGF or other cytokines at least twice as much as smooth muscle cells may be used as the feeder cells of the present invention, but this is not the sole case of the present invention.

In another preferred embodiment, a cell population that contains cells that secrete at least 800 nmol/ml (more preferably about 1500 nmol/ml) of VEGF may be used as the feeder cells of the present invention. To the best knowledge of the present inventors, no report has ever been made of a cell population that contains cells that secrete such a significantly large amount of VEGF and the present inventors are also unaware of the finding that a cell population that contains cells that secrete such a significantly large amount of VEGF can be used as feeder cells; hence, it can be said that this is one of the marked advantages of the present invention.

The feeder cells of the present invention can support any kinds of cells and, hence, they can support any cells that need to be supported. In a preferred embodiment, such cells that should be supported include, but are not limited to, embryonic stem cells, tissue stem cells (e.g. epithelial stem cells such as corneal epithelial stem cells and oral mucosal epithelial stem cells), or differentiated cells.

In one preferred embodiment, the feeder cells of the present invention are used to maintain the cornea. To date, there has been no finding that the cornea can be cultured *ex vivo* for regeneration and the fact that cells that are collectable from adipose tissue can be used as feeder cells may well be considered to be a special beneficiary effect.

In one preferred embodiment, the feeder cells to be used in the present invention preferably contain primary cultured cells. Although not wishing to be bound by any theory, there is no denial that cell lines may potentially cause immunological rejection and advantages of the present invention include, but are not limited to, the fact that autologous cells can be applied in primary cultures. Such cells are preferably primary cultured cells in the strict sense of the term; however, subcultures of cells, if the passage number is small, can also be advantageously used in the present invention since they can have almost the same nature as primary cultured cells. Maximum passage numbers that can be tolerated include, but are not limited to, five passages or less, four passages or less, three passages or less, two passages or less, and one passage.

In the case of humans, fibroblasts into which ES cells had been induced to differentiate have been reportedly used as feeder cells, with a recognized feeder effect. On the other hand, it has only been reported that the prior art using adult cells is not all effective or effective only weakly (Rheinwald JG, Green H., Cell 1975;6:331-344). Therefore, one beneficiary effect that is absent from the prior art and which yet can be achieved by the present invention in one embodiment thereof is that high efficacy could be exhibited using adult cells. It should also be noted that fibroblasts derived from adult fat appear to be more effective than fibroblasts derived from tissues other than adult fat tissue.

In one preferred embodiment, the feeder cells to be used in the present invention contain human cells, and it is particularly preferred for the feeder cells to comprise human cells. In the prior art, established mouse cell lines (e.g. STO cells) or primary cultured fibroblasts have been used as feeder and this has also been the case for humans; therefore, it should be understood that one needs to note that the present invention brings about the advantage of markedly reducing the risk of contamination and infection that might originate from other animals (in particular, in the case of using autologous cells) in regenerative medical treatment in humans.

In a preferred embodiment, the feeder cells of the present invention are advantageously administered antibiotics such as mitomycin C, exposed to radiation, or otherwise treated. This is because by treating or irradiating the feeder cells with a view to stopping their growth, any adverse effects on the cells for which the feeder effect is expected can be prevented.

It has been shown that the feeder cells of the present invention have greater ability than the conventionally used NIH/3T3 cells (particularly in terms of average colony area or the percent dish area as occupied by colonies). Therefore, the present invention also brings about the advantage of providing the feeder ability that has been impossible to attain using the conventional feeder cells.

### (Method of regenerative medical treatment)

In another aspect, the present invention provides a method for preparing a transplant for regenerating an organ, tissue or cell of a subject, comprising the steps of A) providing part of a desired organ, tissue or cell or a stem cell capable of differentiation thereinto; and B) culturing the part or the stem cell on a feeder cell comprising an adipose tissue derived cell. As used herein, part of a desired organ, tissue or cell may be a cell or it may be a tissue section or the tissue itself, or the organ itself. Since regeneration is what is finally desired, such "part" is preferably the normal organ, tissue or cell that is deficient of a portion. As used herein, the stem cell may be any stem cell as long as it can differentiate into a desired organ, tissue or cell. Therefore, embryonic stem cells can be one preferred embodiment of the present invention since it has totipotency. If stem cells are to be used, they are preferably cultured in a culture medium that contains a factor and/or nutrient that promotes differentiation into a desired organ, tissue or cell. Such differentiation-promoting factor varies with the desired tissue, organ, or the like. Examples of such factor include, but are not limited to, cellular physiologically active substances (e.g. growth factors, cytokines, and extracellular matrices).

Specific examples of such factors include, but are not limited to:
A) epidermal growth factor (EGF) for the cornea; B) TGF-beta, FGF-7 (keratinocyte growth factor for KGF) and EGF for the skin (keratinocyte); C) VEGF, FGF and angiopoietin for the vascular endothelium; D) LIF, BMP, FGF and GDNF for the kidney; E) HGF, LIF and VEGF for the heart; F) HGF, TGF-beta, IL-6, EGF and VEGF for the liver; G) VEGF for the umbilical endothelium; H) EGF, IGF-I, HGF, KGF, TGF-beta and IL-11 for the intestinal epithelium: I) nerve growth factor (NGF) and BDNF (brain-derived neurotrophic factor), GDNF (glial cell-line derived neurotrophic factor), neurotrophin, IL-6, TGF-beta and TNF for the nerve; J) TGF-beta, TNF-alpha, EGF, LIF and IL-6 for glial cells; K) bFGF, LIF, TGF-beta, IL-6 and VEGF for peripheral neurons: L) TGF-beta, IL-13, IL-lbeta, KGF and HGF for the lung (pulmonary alveolus epithelium); M) growth hormone (GH), IGF, prolactin, LIF, IL-1, activin A and EGF for the placenta; N) growth hormone and prolactin for the pancreatic epithelium; O) TGF-beta, IGF, PDGF, EGF, TGF-beta and TRH (thyrotropin-releasing hormone) for pancreatic Langerhans cells; P) FGF and TGF-beta for the epithelium of the synovial membrane of a joint; Q) BMP and FGF for osteoblasts; R) FGF, TGF-beta, BMP and TNF-alpha for chondroblasts; S) FGF, CNTF (ciliary neurotrophic factor) for retinal cells; T) insulin, IGF and LIF for adipose cells; U) LIF, TNF-alpha and FGF for muscle cells.

In the method of the present invention, typical examples of the desired organ, tissue or cell include, but are not limited to, epidermic organs, tissues or cells. Specific examples of such desired organ, tissue or cell include, but are not limited to, the cornea, bone, muscle, cartilage, heart, pericardium, blood vessel, skin, kidney, liver, umbilical cord, intestine, nerve, lung, placenta, pancreas, brain, joint, peripheral limbs, fat, retina, and parts thereof. As the most preferred example, the cornea may be mentioned.

The feeder cells to be used in the regenerative medical treatment method of the present invention can be such feeder cells as are described above under "Feeder cells" and they may include stem cells and fibroblasts. Alternatively, the feeder cells to be used in the present invention can be primary cultured cells. Such primary cultured cells may be of any origin but they are preferably cells of the same species; more preferably, syngeneic cells are advantageous and most preferred are autologous cells. This is because immunological rejection can be eliminated by using syngeneic or autologous cells.

In the regenerative medical treatment method of the present invention, human cells are preferably used. This is because the use of human cells as feeder cells has only been reported for cell lines, but not for primary cultured cells. The regenerative medical treatment method of the present invention has a heretofore unaccomplished advantage in that medical treatment is possible on a human subject without using xenogeneic cells. This is because the FDA directives require that a tissue transplanted using xenogeneic cells as feeder cells should be clinically examined for 20 years; such medical treatment involves a huge amount of labor and yet it is persistently accompanied by the concern of immunological rejection. The method of the present invention overcomes this defect when it is applied to a human subject, so it offers an advantage that has been unattainable by the prior art.

Another advantage of the present invention is that it can provide supply sources from adipose tissue. It should be noted that excision from adipose tissue offers many advantages including non-stress collection, as well as excisability from outpatients, repeatability, and large yield.

The regenerative medical treatment method of the present invention is performed *ex vivo.* Since *ex vivo* medical treatment assumes that autologous cells are eventually returned into the subject, it gives the best result from the viewpoint of biocompatibility.

In the regenerative medical treatment method of the present invention, part of a desired organ, tissue or cell or a stem cell that can differentiate into the part and the feeder cell may be xenogeneic, allogeneic or syngeneic to each other as long as the feeder cell exhibits the supportive function; preferably, they are syngeneic, and more preferably they are autologous. Although not wishing to be bound by any theory, immunological rejection can be suppressed if the part or stem cell and the feeder cell are syngeneic. However, if rejection is anticipated, the step of avoiding such rejection may be further included. The procedure of avoiding rejection is known in the art and may be found in "New Encyclopedic Course in Surgery", Vol. 12, "Heart and lung transplantation -- From technological and ethical considerations toward implementation)" (Revised 3rd ed.), Nakayama Publishing Company (in Japanese). Exemplary methods include the use of immunosuppressive drugs and steroids. Immunosuppressive drugs currently used to prevent rejection include cyclosporine (SANDIMMUNE/NEORAL), tacrolimus (PROGRAF), azathioprine (IMURAN), steroid hormones (predonine and methylpredonine), T-cell antibodies (e.g. OKT3 and ATG), and the method being practiced at many facilities in the world for preventive immunosuppressive therapy is the combination of cyclosporine, azathioprine and steroid hormone. It is desirable but not essential that immunosuppressive drugs be administered concurrently with the combination therapy of the present invention. Therefore, as long as the immunosuppressive effect is achieved, immunosuppressive drugs may be administered before or after the combination therapy of the present invention.

In one preferred embodiment, part of a desired organ, tissue or cell or a stem cell that can differentiate into the part is either what has been just excised from the subject or what has been stored frozen. It is preferred to use what has been just excised. As used herein, the term "just" means the time it usually takes to prepare a cell from the excised part or stem cell. Hence, that time may be exemplified by one hour, two hours or three hours but it should be understood that a shorter or longer time may be employed. Part of a desired organ, tissue or cell or a stem cell that can differentiate into the part may be stored frozen and this can be achieved by any methods known in the art. Exemplary methods of storage include, but are not limited to, storage in a solution containing DMSO.

In one embodiment, the conditions for culturing in the regenerative medical treatment method of the present invention and the conditions for culturing part of a desired organ, tissue or cell that is to be used in the present invention or a stem cell that can differentiate into the part may be any conditions that are conventionally used to culture the cell, tissue or organ. Exemplary culture conditions that can be employed are ordinary ones comprising 37 °C and 5% CO₂. The medium to be used may also be of any type and an exemplary medium that can be employed contains DMEM/Ham 12 (1:1), 10% FCS, insulin or cholera toxin. Depending on the need, a culture medium preliminarily supplemented with a differentiating factor (e.g. EGF (10 ng/ml)) may be used but this is not the sole case of the present invention.

In one embodiment, culture in the regenerative medical treatment method of the present invention is advantageously performed in such a way that the ratio between the part of a desired organ, tissue or cell or the stem cell that can differentiate into the part and the feeder cell is in the range of 10:1 to 1:10, preferably from 7:1 to 1:7, and more preferably from 5:1 to 1:5. Although not wishing to be bound by any theory, it appears advantageous that the feeder cell is used in a smaller amount than the part or the stem cell.

In one preferred embodiment, a differentiating factor may be added to effect culture in the method of the present invention. Typical examples of this factor include, but are not limited to, DNA demethylating agents (e.g. 5-azacytidine), histone deacetylating agents (e.g. trichostatin), nuclear receptor ligands (e.g. retinoic acid (ATRA), vitamin D₃ and T3), cell growth factors (e.g. activin, IGF-1, FGF, FDGF, TGF-beta, and BMP2/4), cytokines (e.g. LIF, IL-2, and IL-6), hexamethylene bisacetamide, dimethylacetamide, dibutyl CAMP, dimethyl sulfoxide, iododeoxyuridine, hydroxylurea, cytosine arabinoside, mitomycin C, sodium butyrate, aphidicolin, fluorodeoxyuridine, polybrene, and selenium. For differentiation into the cornea, the differentiating factor EGF may be added. For other differentiated cells, too, any of the differentiating factors described herein may be used.

In another aspect, the present invention provides a method for regenerating an organ, tissue or cell of a subject. This method comprises the steps of A) providing part of a desired organ, tissue or cell or a stem cell capable of differentiation thereinto, B) culturing the part or the stem cell on a feeder cell comprising an adipose tissue derived cell, and C) transplanting the cultured part or stem cell to a site to be treated of the subject. In the process, step A) and step B) can be performed in any of the manners described above in the section of the Regenerative Medical Treatment Method.

Administration can also be performed by any of the methods known in the art. Exemplary methods include, but are not limited to, injection with a syringe, a catheter, a tube and the like. Preferably, local injection (subcutaneous injection or injection into an organ such as muscle or fat), intravenous injection, intraarterial injection, injection over tissue, and the like.

Transplantation to a site to be treated may be direct or indirect. Hence, the graft prepared by a method comprising steps A) and B) may be directly transplanted to the site to be treated; alternatively, the graft may first be transplanted to any other site in the subject and then an *in vivo* delivery system is employed to transplant the graft to the site to be treated. Any suitable manner can be chosen as appropriate by a skilled artisan. Direct transplantation is preferred. Particularly in the case where administration to an accurate place is required as on the cornea, direct transplantation is desired.

In a preferred embodiment, it is advantageous for the method of the present invention to further include mitomycin C administration and irradiation. This is because a change to a carcinoma can be suppressed or an unwanted abnormal cell growth can be prevented by antibiotics addition or irradiation with a view to stopping the growth of the feeder cell.

### (Regenerative medical treatment system)

In yet another aspect, the present invention provides a system for regenerating an organ, tissue or cell of a subject. This system comprises A) a vessel and B) a feeder cell comprising an adipose tissue derived cell. As used herein, the vessel may be of any type as long as it allows the feeder cell to adhere and it can accommodate the cells to be regenerated on the feeder cell. Hence, any vessel can be used. Preferably, the vessel to be used is desirably made of biocompatible materials but it should be understood that any kinds of materials can be used unless they present toxicity to living bodies. Examples of materials of which the vessel can be made include organic materials such as polyethylene, ethylene, polypropylene, polyisobutylene, polyethylene terephthalate, unsaturated polyesters, fluorine-containing resins, polyvinyl chloride, polyvinylidene chloride, polyvinyl acetate, polyvinyl alcohol, polyvinyl acetal, acrylic resins, polyacrylonitrile, polystyrene, acetal resins, polycarbonates, polyamides, phenolic resins, urea resins, epoxy resins, melamine resins, styrene/acrylonitrile copolymer, acrylonitrile/butadiene/styrene copolymer, silicone resins, polyphenylene oxide and polysulfone, as well as materials coated with silane, poly-L-lysine coat, etc.

The feeder cell to be used in the regenerative medical treatment system of the present invention may be any feeder cell as long as it is the above-described feeder cell of the present invention.

It is advantageous for the system to further comprise a providing means for providing part of a desired organ, tissue or cell or a stem cell capable of differentiation thereinto (the providing means may be called a stem cell collecting device). The providing means may be of any type as long as it can provide part of a desired organ, tissue or cell or a stem cell capable of differentiation thereinto. Examples include any means for recovering cells from the subject (e.g. a catheter, a scraping rod, forceps, a syringe, medical scissors, and an endoscope but it should be understood that these are not the sole examples that can be used in the present invention.

In a preferred embodiment, the system of the present invention may further comprise a differentiating factor. Examples of this differentiating factor include, but are not limited to, DNA demethylating agents (e.g. 5-azacytidine), histone deacetylating agents (e.g. trichostatin), nuclear receptor ligands (e.g. retinoic acid (ATRA), vitamin D₃ and T3), cell growth factors (e.g. activin, IGF-1, FGF, PDGF, TGF-beta, and BMP2/4), cytokines (e.g. LIF, IL-2, and IL-6), hexamethylene bisacetamide, dimethylacetamide, dibutyl cAMP, dimethyl sulfoxide, iododeoxyuridine, hydroxylurea, cytosine arabinoside, mitomycin C, sodium butyrate, aphidicolin, fluorodeoxyuridine, polybrene, and selenium. The differentiating factor may be provided in powder form or as a solution. Alternatively, it may be mixed with other components of medium or it may be provided alone.

In a preferred embodiment, it is advantageous for the system of the present invention to further include a source of mitomycin C or a radiation. This is because mitomycin C can be added or irradiation can be applied with a view to stopping the growth of the feeder cell.

### (Co-grafting)

In another aspect, the present invention provides another method for regenerating an organ, tissue or cell of a subject. This method comprises the step of A) transplanting part of a desired organ, tissue or cell or a stem cell capable of differentiation thereinto and a feeder cell comprising an adipose tissue derived cell to a site to be treated of the subject. This method of co-grafting can also be accomplished by the beneficiary effect brought about by the feeder cell of the present invention. As used herein, the feeder cell and the stem cell may be any of the types that are used in the above-described method of regeneration. As used herein, the stem cell and the feeder cell can be mixed in any desired ratio but preferably it can be advantageous for the stem cell to be used in a larger amount than the feeder cell. The stem cell and the feeder cell may be transplanted simultaneously or one may be transplanted earlier than the other.

In a preferred embodiment, it is advantageous for the method of the present invention to further include a treatment with an antibiotic such as mitomycin C or exposure to a radiation. This is because an unwanted abnormal cell growth or a change to a carcinoma can be prevented.

In yet another aspect, the present invention provides a pharmaceutical composition for regenerating an organ, tissue or cell of a subject, comprising A) part of a desired organ, tissue or cell or a stem cell capable of differentiation thereinto and a feeder cell comprising an adipose tissue derived cell. As used herein, the feeder cell and the stem cell may be any of the types that are used in the above-described method of regeneration.

### (Use of adipose tissue derived cells)

In another aspect, the present invention provides use of an adipose tissue derived cell as a feeder cell. In yet another aspect, the present invention provides use of an adipose tissue derived cell for producing a pharmaceutical composition containing a feeder cell. In still another aspect, the present invention provides use of an adipose tissue derived cell for producing a pharmaceutical composition for regenerating an organ, tissue or cell of a subject. As used herein, the feeder cell may be of any type as long as it is the feeder cell of the present invention and its description is given in the above section of "Feeder cells", which may be incorporated herein by reference.

### (Use of human fibroblasts)

In one aspect, the present invention provides primary cultured human fibroblasts for use as feeder cells. Heretofore, there has existed no case of using primary cultures human fibroblasts as feeder cells and, hence, the present invention offers a significant advantage in that primary cultured cells that permit autologous treatment in the human body can be used as feeder cells. As used herein, primary cultured cells usually include, but are not limited to, cultures of up to five passages.

In another aspect, the present invention provides a method for preparing a transplant for regenerating an organ, tissue or cell of a subject. This method comprises the steps of A) providing part of a desired organ, tissue or cell or a stem cell capable of differentiation thereinto and B) culturing the part or the stem cell on a feeder cell comprising primary cultured human fibroblasts. The feeder cell may be any cell as long as it is primary cultured human fibroblasts.

In another aspect, the present invention provides a system for regenerating an organ, tissue or cell of a subject. This system comprises A) a vessel and B) a feeder cell comprising primary cultured human fibroblasts. Depending on the need, the system may further include a stem cell collecting means, a differentiating factor, and the like. Such vessel, stem cell collecting means, differentiating factor and the like may be selected from among those which have been described hereinabove.

In another aspect, the present invention provides a method for regenerating an organ, tissue or cell of a subject. This method comprises the steps of A) providing part of a desired organ, tissue or cell or a stem cell capable of differentiation thereinto, B) culturing the part or the stem cell on a feeder cell comprising primary cultured human fibroblasts, and C) transplanting the cultured part or stem cell to a site to be treated of the subject. As used herein, the providing of the stem cell is described elsewhere in the specification. As described above, the primary cultured fibroblasts to be used may be of any type but those that have not undergone any passage are preferably employed.

In another aspect, the present invention provides another method for regenerating an organ, tissue or cell of a subject. This method comprises the step of A) transplanting part of a desired organ, tissue or cell or a stem cell capable of differentiation thereinto and a feeder cell comprising primary cultured human fibroblasts to a site to be treated of the subject. A detailed form of the stem cell that can be employed herein may be of any type that is described elsewhere in the specification. As described above, the primary cultured fibroblasts to be used may be of any type but those that have not undergone any passage are preferably employed.

In another aspect, the present invention provides a pharmaceutical composition for regenerating an organ, tissue or cell of a subject, comprising part of a desired organ, tissue or cell or a stem cell capable of differentiation thereinto and a feeder cell comprising primary cultured human fibroblasts. A detailed form of the stem cell that can be employed herein may be of any type that is described elsewhere in the specification. As described above, the primary cultured fibroblasts to be used may be of any type but those that have not undergone any passage are preferably employed.

In another aspect, the present invention provides use of primary cultured human fibroblasts as feeder cells, In another aspect, the present invention provides use of primary cultured human fibroblasts for producing a pharmaceutical composition containing feeder cells. In still another aspect, the present invention provides use of primary cultured human fibroblasts for producing a pharmaceutical composition for regenerating an organ, tissue or cell of a subject. Methods of these uses are as described hereinabove and as illustrated in the examples that are given later.

### (Graft for regenerative medical treatment)

In one aspect, the present invention provides a graft for regenerating an epithelial tissue which contains a stem cell or a cell derived from the stem cell. This graft is useful as a means of medical treatment that is really a breakthrough in that it can regenerate an epithelial tissue.

In one embodiment, the epithelial tissue to be regenerated by the present invention can be cornea. This is a great advantage since no method has been available to date that can treat the cornea and which can also improve the visual acuity up to, for example, at least 0.5 or 0.7. In addition, the ability to use autologous cells makes the present invention noteworthy as providing a medical treatment method that is substantially free of rejection.

The stem cell contained in the graft of the present invention may be exemplified by an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell, a hepatic stem cell, and the like, with the epithelial stem cell being preferred. Examples of the epithelial stem cell include, but are not limited to, cells such as a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, a gallbladder epithelial stem cell, and the like.

In one embodiment, the stem cell or the cell derived from the stem cell that are used in the present invention may be a stem cell or a cell that have been co-cultured with a feeder cell. The feeder cell may be a conventionally used feeder cell such as NIH/3T3 but it is also possible to use an adipose tissue derived feeder cell that is provided elsewhere in the specification. It is preferred to use an adipose tissue derived feeder cell. Firstly, autologous cells can be used as feeder cells and secondly, adipose cells are not essential for maintenance of life but rather they might be unwanted for modern people; hence, it can be said that the present invention provides a medical treatment method that is suited to modern people in that unwanted cells can be used to regenerate necessary cells.

Therefore, in one preferred embodiment, the feeder cell to be used in preparing the graft of the present invention preferably comprises an adipose tissue derived cell.

In a preferred embodiment, co-oulturing with the feeder cell is performed under conditions that promote cell adhesion. Such conditions that promote cell adhesion include, for example, the use of a serum-containing culture medium at 37 °C and coating the bottom of a culture dish with various cell adhesion promoting substances such as laminin and fibronectin.

In one embodiment, the graft of the present invention comprises layered cells. Such layering can be accomplished by, for example, using a temperature-sensitive culture dish. This is not the only example and other applicable methods include using a system for culturing epithelial stem cells on a support (carrier) such as the amnion or a fibrin gel, a system for using air lift (a technique of culturing cells at the interface between air and a liquid), and a system for using a culture insert.

In a preferred embodiment, the graft of the present invention is used in non-suture transplantation. With conventional grafts, particularly with those to be used in corneal transplantation, suture transplantation has been essential. Therefore, it is noteworthy that non-suture transplantation has become possible in the medical treatment of epithelial tissues such as corneal transplantation.

In one embodiment, the graft of the present invention brings about a beneficiary effect in that if fat is collected from the patient, all components of sheets that can be prepared are derived from none other than the patient. The structure and the marker do not vary from the conventional grafts but the present inventors have demonstrated that when fat derived stem cells are used as a feeder, epithelial stem cells are retained within the regenerated tissue. In addition, the present inventors have demonstrated that the epithelial stem cells are also retained even if 3T3 is used as a feeder. Therefore, the graft of the present invention can preferably has a structural feature in that it does not contain any xenogeneic componetn but this is not the sole case of the present invention.

In another aspect, the present invention provides use for preparing a pharmaceutical composition as a graft for regenerating an epithelial tissue, the graft containing a stem cell or a cell derived from the stem cell. As the graft, epithelial tissue and epithelial stem cell that are referred to above, any of the forms that are described hereinabove and illustrated below can be adopted.

In one embodiment, the epithelial tissue that is employed in the use of the graft of the present invention as a medicament can be cornea.

In another embodiment, examples of the stem cell that is employed in the use of the graft of the present invention as a medicament include, but are not limited to, an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell, a hepatic stem cell, and the like; epithelial stem cells are preferred, and exemplary epithelial stem cells include, but are not limited to, a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, a gallbladder epithelial stem cell, and the like.

On the following pages, the present invention is described with reference to examples but the following examples are provided for illustrative purposes only. Therefore, the scope of the present invention is not limited by either the above detailed description of the invention or the examples that are given below but is limited only by the appended claims.

### EXAMPLES

Unless otherwise indicated, all reagents used in the following examples were obtained from Wako Pure Chemicals Industries, Ltd. and Sigma. Animals were kept in accordance with the spirit of being kind to animals in compliance with not only the "Principles of Laboratory Animal Care" prepared by the National Society for Medical Research and the "Guide for the Care and Use of Laboratory Animals" (NIH Publication, No. 86-23, revised 1985) prepared by the Institute of Laboratory Animal Resource and published by the National Institute of Health, but also with the guidelines prescribed by the Faculty of Medicine at Osaka University. When the subject was a human, experiments were conducted after having a prior consent in accordance with the guidelines of the Health, Labor and Welfare Ministry.

### (Example 1: Preparation of adipose tissue derived cells)

In this Example, cells were prepared from the adipose tissue of a human who gave consent to the experiment. The aspirated fat mass was thoroughly washed with physiological saline. After confirming that the aspirated fat had separated into the upper layer and the physiological saline into the lower layer, the latter was discarded; this step was repeated until the physiological saline became almost transparent to the naked eye. In this Example, the step was repeated seven times. To 40 ml of the adipose tissue, the same amount (40 ml) of 0.075% collagenase/PBS (Gibco) was added and the mixture was incubated for one hour at 37 °C with thorough agitation. To the sample, an equal volume of 10% serum supplemented DMEM was added and the sample was centrifuged at 1200g for 10 minutes.

To the pellet obtained by centrifugation, 0.16 M NH₄Cl/PBS (Gibco) was added to form a suspension, which was incubated at 25 °C for 20 minutes. The sample was filtered by suction through a screen (Whatman) having openings of 100 µm. The filtrate was centrifuged at 1200g for 5 minutes. The obtained cells were fibroblast-like cells.

### (Example 2: Confirmation of stem cells in the adipose tissue derived cells)

The presence of stem cells in the cells prepared in Example 1 was confirmed by cell markers (CD 105, CD 73, CD 29, CD 44 and Sca-1). The presence of cell markers can be identified by using an immunochemical technique such as ELISA or western blotting.

In this Example, the expression of mRNA was confirmed using pleiotrophin, epiregulin, hepatocyte growth factor, keratinocyte growth factor, sonic hedgehog, Insulin-like growth factor la, and GAPDH. An outline of the experiment is shown below.

Using mRNA that was extracted from cells in a regenerated tissue prepared using adipose derived stem cells as a feeder, cDNA was synthesized. Using the synthesized cDNA as a template, PCR was performed with the primers specific to each of the genes mentioned above and the expression of each gene in the regenerated tissue was checked.

The primers (sense and antisense primers) used for the respective genes are indicated below: pleiotrophin's sequence information: SEQ ID NOS. 1-2; epiregulin's sequence information: SEQ ID NOS. 3-4; hepatocyte growth factor's sequence information: SEQ ID NOS. 5-6; keratinocyte growth factor's sequence information: SEQ ID NOS. 7-8: sonic hedgehog's sequence information: SEQ ID NOS. 9-10: insulin-like growth factor la's sequence information: SEQ ID NOS. 11-12; and GAPDH's sequence information: SEQ ID NOS. 13-14.

The results are shown in Fig. 1B. For the proteins and the like which would affect the growth and maintenance of epithelial cells in NIH/3T3, the expression of mRNA in adipose progenitor cells was analyzed. In particular, cystatin C, HGF, KGF and IGF-la were expressed, suggesting that the adipose progenitor cells would be capable of serving as feeder cells for epithelial cells.

### (Example 3: Characterization of the adipose tissue derived cells)

In the next place, check was made to determine that the cells prepared in Example 1 contained those which had an enhanced ability to secrete cytokines. First, a VEGF sample as secreted from the cells was subjected to ELISA using an anti-VEGF antibody. ELISA was performed using an ELISA kit available from R&D. As controls, human vascular endothelial cells and human derived smooth muscle cells were measured.

As the result, the human vascular endothelial cells were found to have secreted VEGF in an amount of 100 nmol/ml, the human derived smooth muscle cells, about 800 nmol/ml, and the cells prepared in Example 1, about 1500 nmol/ml. Therefore, it was verified that the cells of the present invention had the VEGF secretion enhanced about 2-15 times as much as the ordinary cells. Although not wishing to be bound by any theory, it became clear that the cells of the present invention appeared to have a potentiated feeder effect by containing cells that were enhanced in secretion of cellular physiological substances (e.g. growth factors and cytokines).

### (Example 4: Excision of corneal epithelial stem cells)

In the next place, an attempt was made to regenerate corneal cells using a rabbit model (New Zealand white species; CHARLES RIVER LABORATORIES JAPAN. INC.) First, corneal epithelial stem cells were prepared from the rabbit. The protocol is described below.

Corneal epithelial stem cells were collected from the corneal limbus of a donor's cornea or a host's healthy eye (in the unilateral case). Only a fragment of the corneal limbus was collected at a diameter of 1 mm distant from the center of the cornea, so even if the fragment were collected from a living-related donor's eye or a host's healthy eye, the injury would heal without any scar left and the visual acuity would not be affected at all. After surgery, a therapeutic contact lens was put on the eye, so the wound would heal soon, causing only a slight pain. The post-operative pain was dealt with by administering an analgesic, an antibiotic or an anti-inflammatory agent. It was confirmed that the wound on the rabbit healed at an early stage.

The collected cells were used as the desired stem cells.

### (Example 5: Preparation of oral mucosal stem cells)

In the next place, an attempt was made to regenerate corneal cells using a rabbit model (New Zealand white species; CHARLES RIVER LABORATORIES JAPAN, INC.) In order to try application in a case where the cornea was lost, oral mucosal epithelial stem cells were prepared from the rabbit. The protocol is described below.

Oral mucosal epithelial stem cells were collected from the oral mucosa of a donor's oral mucosa or a host's healthy oral mucosa. Only a fragment with a diameter of 3-5 mm was collected from the buccal mucosa. Since the oral mucosa has high capacity for regeneration, the injury would heal without any scar left and the function of the oral mucosa was not affected at all. Since the oral mucosa is a fast healing tissue, the wound would heal soon, causing only a slight pain. When the oral mucosa was collected from a host, the post-operative pain was dealt with by administering an analgesic, an antibiotic or an anti-inflammatory agent. The wound will usually heal at an early stage.

The collected cells were used as an example of the desired stem cells.

### (Example 6: Confirmation by colony formation assay)

The stem cells prepared in Example 4 were cultured on the feeder cells prepared in Example 1 and a colony formation assay was made to check for the feeder effect. Formation of colonies in this experiment will demonstrate the feeder effect.

The stem cells were cultured on the feeder cells in a medium comprising DMEM/Ham's 12 (1:1; both obtained from Gibco) that was supplemented with 10% fetal calf serum (FCS = obtained from Hana-Nesco Bio, Tokyo), insulin (5 µg/ml = obtained from Sigma), cholera toxin (1 nM = obtained from Wako Pure Chemical Industries, Ltd., Osaka) and EGF (10 ng/ml = obtained from Genzyme, Cambridge, MA). After the culture, the colonies were stained with Rhodamine. A thousand rabbit corneal epithelial cells (primary/8-wk old) were sown. As a control, stem cells were cultured using 3T3 as a feeder. The amount of each type of feeder cells was as follows: 2 x 10⁴ cells for 3T3; 1 x 10⁴ for deep tissue derived cells; and 1 x 10⁴ for subcutaneous tissue derived cells (µg/ml in the panels indicates the concentration of mitomycin C).

The results are shown in Figs. 2-4. Fig. 2 shows the case of using 3T3 cells, Fig. 3 the case of using the deep tissue derived cells, and Fig. 4 the case of using the subcutaneous adipose tissue derived cells. The adipose derived cells of the present invention were found to be capable of forming colonies similar to those formed by using the conventional 3T3. It was thus confirmed that the adipose tissue derived cells of the present invention could be used as feeder cells.

The stem cells prepared in Example 5 were also confirmed to have the same feeder effect.

After irradiating NIH/3T3 and adipose precursor cells with X-rays (20 Gy) under another condition, one-day culture was effected, followed by trypsin treatment to prepare feeder cells; then human corneal epithelial cells were seeded at a density of 1000 cells/dish; at 14 days of the culture, the cells were fixed with formalin, and then stained with rhodamine B; compared with NIH/3T3 feeder, the adipose precursor cell feeder enabled larger colonies to be observed (Fig. 4B).

In the next place, the percent colony formation of these cells, the average area of colonies, and the percentage of the overall dish area occupied by the area of colonies were determined.

Among colonies formed by culture conducted on NIH/3T3 feeder and the adipose precursor cell feeder, the number of colonies larger than 5 mm in diameter was counted visually; the percent colony formation was calculated, with the number of seeded cells (1000) put as a denominator and the number of >5 mm colonies as a numerator: the percent colony formation from NIH/3T3 feeder was about twice that from the adipose precursor cell feeder (Fig. 4C).

The average area of colonies was determined by the following procedure. The size of colonies formed by culture conducted on NIH/3T3 feeder and the adipose precursor cell feeder was measured using NIH image; the average colony size from the adipose feeder was about 2.4 times as large as from NIH/3T3 (Fig. 4D).

The percentage of the overall dish area occupied by the area of colonies was determined by the following procedure. The proportion of the dish area occupied by the area of colonies (total colony area/dish area) was calculated; since the size of individual colonies was larger in the case of the adipose precursor cell feeder, the percentage of the dish occupied by the area of colonies from the adipose precursor cell feeder was about 1.5 times the value from NIH/3T3 feeder (Fig. 4E).

### (Example 7: Preparation of a feeder cell system)

In the next place, there was constructed a system in which the cells prepared in Example 1 were used as feeder cells. These cells were sown on a temperature-responsive dish. Regeneration was effected using this feeder system. The temperature-responsive culture dish was prepared in the following manner. A temperature-responsive intelligent polymer (N-isopropyl acrylamide) was exposed to an electron beam so that it was fixed to a culture dish by covalent bonding. In this Example, the polymer was obtained from CellSeed Inc. (http://www.cellseed.com/; Tokyo, Japan). As one can see, this is how the feeder cell system of the present invention is constructed.

### (Example 8: Differentiation of corneal epithelial stem cells)

Using the system prepared in Example 7, corneal epithelial stem cells were cultured at 37 °C for 2 weeks, There was used a culture medium having the same composition as used in Example 6. A schematic diagram for the culture is shown in Fig. 1. Although the use of a temperature-sensitive culture dish is shown as an example, it should be understood that the present invention can be implemented without using such a dish.

First, 3T3 cells and the cells prepared in Example 1 were treated with mitomycin to be deprived of the growth activity and then seeded for culture on a culture dish coated with a temperature-responsive intelligent polymer.

In the next place, dispase was allowed to act on the collected fragments of the corneal limbus and the oral mucosa so that the epithelial layer would be detached from the basement membrane. Subsequently, a trypsin/EDTA solution (Clontech) was applied to disintegrate the cell layer into individual cells.

The cells were then seeded at a density of 1 x 10⁵ cells/ml on a culture dish coated with the temperature-responsive intelligent polymer sown with the mitomycin-treated 3T3 cells (2 x 10⁴ cells/ml) or the mitomycin-treated cells of Example 1 (2 x 10⁴ cells/ml). All steps of culture following the collection of the fragments were performed by a clean operation within a clean bench. Culture was consistently performed in a clean environment.

A sheet was confirmed to form at 7 and 10 days after the start of culture. As a result, it became clear that like the 3T3 cells, the cells prepared in Example 1 had the feeder effect of regenerating rabbit stem cells in sheet form.

### (Example 9: Differentiation of human corneal epithelial stem cells)

In the next place, check was made to see whether human stem cells could actually be regenerated using the same experimental system as prepared in Example 8. First, the method of cell collection from a human is described below.

Corneal epithelial stem cells were collected from the corneal limbus of a donor's cornea or a host's healthy eye (in the unilateral case). Only a fragment of the corneal limbus was collected at a diameter of 1 mm distant from the center of the cornea, so even if the fragment were collected from a living-related donor's eye or a host's healthy eye, the injury would heal without any scar left and the visual acuity would not be affected at all. After surgery, a therapeutic contact lens was put on the eye, so the wound would heal soon, causing only a slight pain. The post-operative pain was dealt with by administering an analgesic, an antibiotic or an anti-inflammatory agent. It was confirmed that the wound on the rabbit healed at an early stage. The patient had given informed consent about the site and size of tissue collection and the pain after surgery.

As in Example 8, 3T3 cells and the cells prepared in Example 1 were treated with mitomycin to be deprived of the growth activity and then seeded for culture on a culture dish coated with a temperature-responsive intelligent polymer.

In the next place, dispase was allowed to act on the collected fragments of the corneal limbus and the oral mucosa so that the epithelial layer would be detached from the basement membrane. Subsequently, a trypsin/EDTA solution (Clontech) was applied to disintegrate the cell layer into Individual cells.

The cells were then seeded at a density of 1 x 10⁵ cells/ml on a culture dish coated with the temperature-responsive intelligent polymer sown with the mitomycin-treated 3T3 cells (2 x 10⁴ cells/ml) or the mitomycin-treated cells of Example 1 (2 x 10⁴ cells/ml). All steps of culture following the collection of the fragments were performed by a clean operation within a clean bench. Culture was consistently performed in a clean environment. In Example 9, there was used a culture medium of the same composition as used in Example 6 and this culture medium and the bovine serum to be added to it were both of a prion-free and traceable Australian origin (and obtained from Hana-Nesco Bio, Tokyo).

A sheet was confirmed to form at 7 and 10 days after the start of culture. The results are shown in Fig. 5, from which one can clearly see the differentiation of the human corneal epithelial cells into cells that resembled corneal cells. The process of differentiation was not very much different between 3T3 and the human fibroblasts, clearly indicating the sufficient feeder effect of the primary cultured human fibroblasts. It was therefore clear that like the 3T3 cells, the cells of the present invention also had the feeder effect in the case of regenerating human corneal stem cells in sheet form.

The results are shown in Fig. 6. X-ray treated NIH/3T3 and adipose precursor cells were used as feeders in culturing human corneal epithelial cells; 35 mm dishes (coated with NIPAAm) were seeded with corneal epithelial cells at a density of 1 x 10⁵ cells/dish, which were peeled off at 14 days of culture to recover corneal epithelial cell sheets.

The corneal epithelial cells cultured using the adipose precursor cell feeder formed a checkerboard pattern which was by no means inferior to the corneal epithelial cells cultured using the NIH/3T3 feeder.

The sheet was subjected to tissue staining. In order to observe the fixation and subsequent behavior of the support in the cells, hematoxylin-eosin (HE) stain was conducted by the following procedure. After optional deparaffinization (by, for example, pure ethanol) and water rinse, the sample was immersed in Omni hematoxylin for 10 minutes. Thereafter, the sample was rinsed under running water and treated with aqueous ammonia for 30 seconds to allow stain to develop. Thereafter, the sample was rinsed under running water for 5 minutes and stained with a 10-fold dilution of eosin hydrochloride for 2 minutes, followed by dehydration, clearing, and embedding.

### (Example 10: Differentiation of human oral epithelial stem cells)

In the next place, check was made to determine that oral mucosal cells were also regenerated in sheet form.

Oral mucosal epithelial stem cells were collected from the oral mucosa of a donor's oral mucosa or a host's healthy oral mucosa. Only a fragment with a diameter of 3-5 mm was collected from the buccal mucosa. Since the oral mucosa has high capacity for regeneration, the injury would heal without any scar left and the function of the oral mucosa was not affected at all. Since the oral mucosa is a fast healing tissue, the wound would heal soon, causing only a slight pain. When the oral mucosa was collected from a host, the post-operative pain was dealt with by administering an analgesic, an antibiotic or an anti-inflammatory agent. The wound will usually heal at an early stage. The patient had given informed consent about the site and size of tissue collection and the pain after surgery.

The other aspects of the procedure were in accordance with Example 9. As the result, it become clear that like the 3T3 cells, the cells of the present invention also had the feeder effect in the case of regenerating the stem cells of human oral mucosal cells in sheet form.

### (Example 11: Transplanting a differentiated tissue of rabbit corneal epithelial stem cells)

In the next place, the sheet prepared in Example 8 was transplanted into a rabbit. In Example 11, an aggregation of differentiated cells was transplanted onto an actual cornea and checked for the occurrence of engraftment.

A tissue sheet was prepared over a period of 2 weeks as in Example 8 and, prior to the use of a cultured corneal epithelium, the sheet was washed with a serum-free solution and then transplanted. Immediately before transplantation, a bacterial and fungal culture test was conducted to determine that there was no contamination with bacteria or fungi and only after that, the cultured corneal epithelium was used. If desired, a single donor's cornea may be applied to more than one patient.

Two weeks later, the cell aggregation in sheet form was separated by lowering the temperature. Engraftment was examined every week by checking for inflammation, calcification, immunological rejection and other effects. As it turned out, the cell aggregation was engrafted without any significant deleterious actions even after the passage of a certain period of time.

### (Example 12: Regenerative medical treatment in humantransplanting a differentiated tissue of human corneal epithelial stem cells)

The sheet prepared in Example 9 was transplanted into a human patient. In Example 12, an aggregation of differentiated cells was transplanted onto an actual cornea and checked for the occurrence of engraftment. The patient had given informed consent about the method of treatment, the pain after surgery, and potential side effects.

Any experiment on humans require the following minimum precautions:
(1) perform under local or general anesthesia;
(2) remove as much cicatricial tissue from the cornea and conjunctiva as possible before the cultured epithelial sheet is transplanted;
(3) perform pre- and post-operative management as in corneal grafting ("Guidance for Corneal Grafting -- from indications to post-operative management" (in Japanese), Tsubota et al., Nankodo, 2002).

A tissue sheet was prepared over a period of 2 weeks as in Example 8 and, prior to the use of a cultured corneal epithelium, the sheet was washed with a serum-free solution and then transplanted. Immediately before transplantation, a bacterial and fungal culture test was conducted to determine that there was no contamination with bacteria or fungi and only after that, the cultured corneal epithelium was used. If desired, a single donor's cornea may be applied to more than one patient.

Two weeks later, the cell aggregation in sheet form was separated by lowering the temperature. Engraftment was examined every week by checking for inflammation, calcification, immunological rejection and other effects. As it turned out, the cell aggregation was engrafted without any significant deleterious actions even after the passage of a certain period of time.

### (Example 13: Regenerative medical treatment in humanstransplanting a differentiated tissue of human oral mucosal epithelial stem cells)

In the next place, oral mucosal epithelial stem cells were collected from a patient who had given informed consent and the cells were treated as in Example 10 to prepare a graft in sheet form. The graft was transplanted into human subjects: it was thus verified that even the sheet derived from oral mucosal stem cells could make a reasonably functional graft.

In the next place, the sheet was transplanted to the human cornea as in Example 12.

The treated patients were as described below. In the following experiment, 3T3 cells were used as feeder cells.

**Table: Patients Operated Upon**

| Patient | Age | Sex | Eye | Eyeball adhesion | Schirmer test (with topical anesthesia) | Schirmer test (with irritation of nasal cavity) |
|---|---|---|---|---|---|---|
| 1 | 58 | male | right | yes | 1 mm | 2 mm |
| 2 | 69 | male | left | yes | 23 mm | 26 mm |
| 3 | 72 | female | right | yes | 1 mm | 1 mm |
| 4 | 75 | female | right | yes | 1 mm | 2 mm |

| Patient | Diagnostic symptoms, etc. |
|---|---|
| 1 | Stevens-Johnson syndrome (in chronic stage), received allogeneic corneal epithelial grafting (2000) |
| 2 | Cicatricial pemphigoid |
| 3 | Cicatricial pemphigoid, Marginal grafting with amnion (2001). Also had cases of proliferative diabetic retinopathy and obstruction of retinal venous branches. |
| 4 | Cicatricial pemphigoid. Had received corneal grafting (penetrating keratopathy) (2001). |

In the Schirmer test without topical anesthesia, the amount of water that was produced by applying 5 mm x 35 mm Whatman filter paper to the margin for 5 minutes was measured. Values smaller than 5 mm indicate impaired tear secretion.

In the Schirmer test with irritation of the nasal cavity, the amount of water that was produced by irritating the nasal cavity with a cotton swab was measured. Values smaller than 10 mm indicate a decrease in the volume of lacrimal gland.

Figs. 7B-7H are stains of the sheets.

Fig. 7B shows the confirmation by H&E staining that the recovered cell sheet was formed of 3-5 cell layers.

Fig. 7C is an H&E stain of the initial oral mucosa which is entirely different from the cell sheet.

Fig. 7D shows the normal corneal epithelial cells; the cell sheet of the invention is similar to these corneal epithelial cells.

Fig. 7E is a micrograph showing the development of microvilli on the tip surface of the cell sheet to be used in the present invention.

Fig. 7F is a micrograph of the cell sheet to be used in the present invention which was immunologically stained (in green) with anti-keratin 3 antibody.

Fig. 7G is a micrograph of the cell sheet to be used in the present invention which was immunologically stained (in green) with anti-beta 1 integrin antibody.

Fig. 7H is a micrograph of the cell sheet to be used in the present invention which was immunologically stained (in green) with anti-p63 antibody.

Immunological staining was performed using an anti-keratin 3 antibody (AE5, Progen Biotechnik), anti-beta 1 integrin antibody (P5D2, Santa Cruz Biotechnology) and anti-p63 antibody (4A4, Santa Cruz Biotechnology). Also used was a secondary antibody that was labelled with either fluorescein isothiocyanate or Rhodamine (Jackson ImmunoResearch Laboratories). The nucleus was stained with Hoechst 33342 (Molecular Probes) or propidium iodide (Sigma).

The stained cells were examined with a confocal microscope (LSM-510, Zeiss). A sample having nonspecific IgG (of the same concentration) was used as a negative control whereas a native human cornea and a marginal tissue were used as positive controls.

Surgery was initiated by removing a scarred tissue of and under the conjunctiva from the cornea up to 3 mm outside of the margin so that the corneal stroma was exposed. Then, the prepared cell sheet was placed on the exposed area of the cornea. No suture was necessary. The process of transplantation is shown in Fig. 7I. Fig. 7I-A shows the overall surface of the cornea, with some neovascularization occurring in the conjunctiva. Fig. 7I-B shows the conjunctival tissue on the cornea, which was re-exposed to reveal the corneal stroma on the surface. Fig. 7I-C shows how the cell sheet is recovered by means of a doughnut-shaped supporter. Fig. 7I-D shows the cell sheet placed on the corneal stroma. Fig. 7I-E shows the cell sheet that adhered in a few minutes to the corneal stroma, from which the supporter was later detached. Fig. 7I-F shows engraftment of the cell sheet on the corneal stroma.

After the surgery, local treatment was performed using an antibiotic (0.3% ofloxacin) and a steroid (0.1% betamethasone), initially four times a day, then three times a day. In the first one week, betamethasone was administered orally. At one month after the surgery, the local administration of corticosteroid was changed from betamethasone (0.1%) to fluorometholone (0.1%). For a symptom of dry eye had appeared. Artificial tear was also used as required.

The appearance of the eyes of the four patients after surgical operation is shown in Figs. 7J-1 to 7J-4. Numerals 1 to 4 identify the patient numbers in the foregoing table. The left panels show the pre-operative eyes and the right panels show the post-operative eyes.

The recovery of visual acuity after surgery is summarized in the following table, as compared with the pre-operative condition.

**Table: Comparison Between Pre- and Post-operative Conditions of the Patients**

| Patient number | Visual acuity before surgery | Visual acuity after surgery | Corneal opacity before surgery | Corneal opacity one month after surgery | Corneal opacity at the last diagnosis | Complications | Monitor period (months) |
|---|---|---|---|---|---|---|---|
| 1 | could count fingers | 20/100 | 3 | 2 | 1 | none | 15 |
| 2 | 20/2000 | 20/25 | 3 | 1 | 1 | none | 14 |
| 3 | hand movement | 20/300 | 3 | 1 | 1 | none | 14 |
| 4 | 20/2000 | 20/50 | 3 | 1 | 1 | none | 13 |

The corneal opacity was evaluated by the following scale: 0, clear; 1, moderately cloudy: 2 moderately cloudy, with partial hiding of the iris; 3, heavily cloudy, with hiding of the intraocular structure.

If the patients were unable to read the eye chart, their visual acuity was measured by asking them to count the number of fingers. If they were unable to count the number of fingers, they were asked to read a hand's movement. The visual acuities that were measured after best correction are shown in the table.

As the above table shows, the graft of the present invention was used in Example 13 to perform medical treatment of the cornea and it was verified to be effective in the recovery of vision.

Thus, the aggregation of cells into which oral mucosal epithelial stem cells differentiated was also verified to be successfully engrafted after the passage of a certain period of time without causing any significant deleterious action.

### (Example 14: Corneal transplantation using adipose derived cells as feeder cells)

The same protocol was employed to verify that equally effective engraftment and good recovery of vision could be achieved by using adipose derived cells as feeder cells.

The adipose tissue derived cells that were prepared as in Examples 1-3 were used as feeder cells. Oral mucosal stem cells were prepared as described in Example 5 and co-cultured with the feeder cells.

In the co-culture, the cell system described in Example 7 was used. Differentiation was stimulated as described in Example 10.

The cell sheet thus prepared was used in an experiment for transplanting it to the cornea according to the protocol described in Example 13.

As a result, grafting to the cornea could successfully be achieved by using the adipose tissue derived cells of the present invention as feeder cells and among the beneficial effects verified was smaller possibility of rejection than in the case of using 3T3 cells.

### (Example 15: Treatment other than on the corneaepidermis)

This Example intends to confirm that the feeder cells of the present invention can be used to culture the epidermis as effectively as the cornea. It also intends to confirm that even an already commercially available epidermal sheet that is a heterologous graft cultured by the ordinary protocol can be co-cultured with or adhered to autologous adipose tissue derived fibroblasts.

Epidermis was collected by a method known in the art. Specifically, skin was collected from a human patient who had given informed consent. From the collected skin, stem cells were prepared in accordance with the procedure described in Example 9 or 10. By the same technique as employed in Example 9 or 10, the stem cells were verified to have the feeder effect of the cells of the present invention. Using the same technique as employed with the cornea, a sheet was formed of the stem cells on a temperature-responsive dish. It was then confirmed that a sheet could be formed of the stem cells as effectively as in the case of the cornea.

### (Example 16: Effect with embryonic stem cells)

In the next place, an experiment was conducted to confirm that the feeder cells of the present invention exhibit the intended feeder effect even if they are embryonic stem cells. Embryonic stem cells were prepared using such a technique as described in "Protocols of Studies on Stem Cells and Clones" (in Japanese), ed. by Nakatsuji, Yohdosha (2001). By the same procedure as employed in Example 9 or 10, the embryonic stem cells were verified to have the feeder effect of the cells of the present invention. When no differentiating factor is used, it is designed to perform an experiment for achieving an amplifying effect (maintaining and proliferating the cells as they remain undifferentiated). When the embryonic stem cells were used together with a desired differentiating factor, promotion of their differentiation into various organs could also be confirmed.

In the case of using embryonic stem cells, cells preliminarily collected from the adipose tissue stem cells of a recipient may be co-cultured with the ES cells and this increases the likelihood that the recipient will not suffer any immunological rejection against an organ or tissue produced from the ES cells. This can potentially provide immunological tolerance not only in regenerative medicine but also in medical transplantation of cells or an organ into which cells have differentiated.

### (Example 17: Effect with co-grafting)

In the next place, a feeder effect was verified to occur when the feeder cells of the present invention were transplanted into the body together with stem cells.

In this Example, the feeder cells of the present invention could be transplanted into the living body simultaneously with a sheet of epidermal or other stem cells without deactivating those feeder cells by antibiotics or irradiation. By this treatment, one can expect a growth factor secreting action or its reinforcing function to occur within the living body. Since the method of the present invention permits the use of autologous tissues, one can see that side reactions such as rejection are considerably reduced while at the same time the regenerating effect is improved over the prior art level.

### (Example 18: Effect with a human tissue: Feeder effect of the invented feeder cells on human keratinocytes)

Check was made to see whether the adipose tissue derived cells of the present invention would have the feeder effect on human keratinocytes. An assay of human keratinocyte colonies was conducted in the following manner. A system of feeder cells was constructed as shown in Fig. 7 and then used.

After irradiating NIH/3T3 and adipose precursor cells with X-rays (20 Gy), one-day culture was effected, followed by trypsin treatment to prepare feeder cells. Then, human keratinocytes were seeded at a density of 1 x 10⁴ cells/dish. At 14 days of the culture, the cells were fixed with formalin and then stained with rhodamine B (Fig. 8). The adipose precursor cells also showed the feeder effect on the keratinocytes, revealing the possibility of their working as feeder cells for other epithelial cells than corneal epithelial cells.

### (Example 19: Feeder effect of the present invention on vascular endothelial cells)

In the next place, the feeder effect of the present invention on vascular endothelial cells was verified. A system of feeder cells was constructed as shown in Fig. 7 and then used. Angiogenesis kit (Kurabo, Tokyo, Japan) was used.

In order to see if human adipose precursor cells would become feeder cells capable of promoting angiogenesis, the conditioned medium of the adipose precursor cells was added to a 3-dimensional co-culture system of human vascular endothelial cells and fibroblasts (Kurabo), whereupon the lumen forming capability was significantly enhanced in comparison with the conditioned medium of endothelial cells (Fig. 9). Check was also made for the migrating capability of endothelial cells and the conditioned medium of adipose precursor cells showed significant enhancement in comparison with the conditioned medium of endothelial cells (Fig. 9). It was therefore clear that the adipose precursor cells also showed the feeder effect on the human vascular endothelial cells.

### (Example 20: Feeder effect of the feeder cells of the present invention on bone marrow derived mesenchymal stem cells)

In the next place, the feeder effect of adipose precursor cells on human vascular endothelial cells was verified. Comparison was made between a culture medium alone (10% FCS + DMEM) and the culture medium with mouse adipose precursor cells as feeder cells. Bone marrow mononuclear cells were isolated from mouse bone marrow by Histopaque (density-gradient centrifugation method) and seeded on both samples. At 7 days of the culture, the suspended cells were isolated and subjected to May-Giemsa staining. The results are shown in Fig. 10. It became clear that the adipose precursor cells also showed the feeder effect on the bone marrow mononuclear cells.

Although the present invention has been illustrated above by reference to certain of its preferred embodiments, the present invention should not be interpreted as being limited to those embodiments. It is understood that the scope of the present invention should only be interpreted on the basis of the appended claims. Any skilled artisan will understand from the description of the specific preferred embodiments of the present invention that an equivalent scope can be implemented on the basis of the description of the present invention and common technical knowledge. It is also understood that all patents, patent applications and references cited herein should be incorporated herein by reference as is set forth specifically herein.

### INDUSTRIAL APPLICABILITY

The present invention has much utility in regenerative medicine. In preferred embodiments, the present invention finds particularly high utility since it has for the first time accomplished the use of human cells as a feeder. In addition, the present invention uses the abundant resources, i.e. adipose tissue, so it has dissipated the concern over the supply source. In this sense, the present invention may well be described as having high utility. Therefore, the present invention has applicability in regenerative medicine and in the art of manufacturing the necessary therapeutic pharmaceuticals.

## Claims

1. An adipose tissue derived cell for use as a feeder cell.

2. The cell according to claim 1 wherein the feeder cell is for differentiating or maintaining an embryonic stem cell, a tissue stem cell or a differentiated cell.

3. The cell according to claim 1 wherein the feeder cell is for effecting differentiation to or maintenance of epidermis.

4. The cell according to claim 1 wherein the feeder cell is for effecting differentiation to or maintenance of cornea.

5. The cell according to claim 1 wherein the cell comprises a tissue stem cell.

6. The cell according to claim 1 wherein the cell comprises a fibroblast.

7. The cell according to claim 1 wherein the cell comprises a primary cultured cells.

8. The cell according to claim 1 wherein the cell comprises a human cell.

9. A method for preparing a transplant for regenerating an organ, tissue or cell of a subject, comprising the steps of:
A) providing part of a desired organ, tissue or cell or a stem cell capable of differentiation thereinto; and
B) culturing the part or the stem cell on a feeder cell comprising an adipose tissue derived cell.

10. The method according to claim 9 wherein the desired organ, tissue or cell comprises an epidermic one.

11. The method according to claim 9 wherein the desired organ, tissue or cell is selected from the group consisting of cornea, bone, muscle, cartilage, heart, pericardium, blood vessel, skin, kidney, liver, umbilical cord, intestine, nerve, lung, placenta, pancreas, brain, joint, limb peripheries, fat, retina, and parts thereof.

12. The method according to claim 9 wherein the feeder cell comprises a tissue stem cell.

13. The method according to claim 9 wherein the feeder cell comprises a fibroblast.

14. The method according to claim 9 wherein the feeder cell comprises a primary cultured cells.

15. The method according to claim 9 wherein the subject and the feeder cell are derived from the same species.

16. The method according to claim 9 wherein the subject is human and the feeder cell is a human cell.

17. The method according to claim 9 wherein the culturing is performed *ex vivo*.

18. The method according to claim 9 wherein the part or the stem cell and the feeder cell are xenogeneic, allogeneic or syngeneic.

19. The method according to claim 9 wherein the part or the stem cell and the feeder cell are syngeneic.

20. The method according to claim 9 wherein the part or the stem cell is either what has been just excised from the subject or what has been stored frozen.

21. The method according to claim 9 wherein the culturing is performed in the presence of at least one factor selected from the group consisting of fetal calf serum, insulin or cholera toxin.

22. The method according to claim 9 wherein the culturing is performed at the ratio of the part or the stem cell to the feeder cell between the range of 10:1 to 1:10.

23. The method according to claim 9 wherein the culturing is performed using the feeder cell in a smaller amount than the part or the stem cell.

24. The method according to claim 9 wherein the culturing is performed in a culture medium containing a cytophysiologically active substance.

25. The method according to claim 9 wherein the culturing is performed in a culture medium containing an epidermal growth factor (EGF) and wherein the desired organ, tissue or cell contains cornea, its tissue or cell.

26. The method according to claim 9 which further includes the step of suppressing the growth of the feeder cell.

27. The method according to claim 26 wherein the suppressing of the growth of the feeder cell is achieved by an antibiotic administration or an irradiation.

28. The method according to claim 27 wherein the antibiotic comprises mitomycin C.

29. A system for regenerating an organ, tissue or cell of a subject, comprising:
A) a vessel; and
B) a feeder cell comprising an adipose tissue derived cell.

30. The system according to claim 29 which further comprises a providing means for providing part of a desired organ, tissue or cell or a stem capable of differentiation thereinto.

31. The system according to claim 30 wherein the providing means includes a means for recovering the part or the stem cell from the subject.

32. The system according to claim 31 wherein the means includes means selected from the group consisting of a catheter, a scraping rod, forceps, a syringe, medical scissors, and an endoscope.

33. The system according to claim 29 which further comprises
a cytophysiologically active substance.

34. The system according to claim 29 which further comprises EGF.

35. The system according to claim 29 which further includes means for suppressing the growth of the feeder cell.

36. A method for regenerating an organ, tissue or cell of a subject, comprising the steps of:
A) providing part of a desired organ, tissue or cell or a stem cell capable of differentiation thereinto;
B) culturing the part or the stem cell on a feeder cell comprising an adipose tissue derived cell; and
C) transplanting the cultured part or stem cell to a site to be treated of the subject.

37. A method for regenerating an organ, tissue or cell of a subject, comprising the step of:
A) transplanting part of a desired organ, tissue or cell or a stem cell capable of differentiation thereinto and a feeder cell comprising an adipose tissue derived cell to a site to be treated of the subject.

38. A pharmaceutical composition for regenerating an organ, tissue or cell of a subject, comprising:
A) part of a desired organ, tissue or cell or a stem cell capable of differentiation thereinto and a feeder cell comprising an adipose tissue derived cell.

39. Use of an adipose tissue derived cell as a feeder cell.

40. Use of an adipose tissue derived cell for producing a pharmaceutical composition containing a feeder cell.

41. Use of an adipose tissue derived cell for producing a pharmaceutical composition for regenerating an organ, tissue or cell of a subject.

42. A primary cultured human fibroblasts for use as a feeder cell.

43. A method for preparing a transplant for regenerating an organ, tissue or cell of a subject, comprising the steps of:
A) providing part of a desired organ, tissue or cell or a stem cell capable of differentiation thereinto: and
B) culturing the part or the stem cell on a feeder cell comprising a primary cultured human fibroblasts.

44. A system for regenerating an organ, tissue or cell of a subject, comprising:
A) a vessel; and
B) a feeder cell comprising a primary cultured human fibroblasts.

45. A method for regenerating an organ, tissue or cell of a subject, comprising the steps of:
A) providing part of a desired organ, tissue or cell or a stem cell capable of differentiation thereinto;
B) culturing the part or the stem cell on a feeder cell comprising a primary cultured human fibroblasts; and
C) transplanting the cultured part or stem cell to a site to be treated of the subject.

46. A method for regenerating an organ, tissue or cell of a subject, comprising the step of:
A) transplanting part of a desired organ, tissue or cell or a stem cell capable of differentiation thereinto and a feeder cell comprising a primary cultured human fibroblasts to a site to be treated of the subject.

47. A pharmaceutical composition for regenerating an organ, tissue or cell of a subject, comprising:
A) part of a desired organ, tissue or cell or a stem cell capable of differentiation thereinto and a feeder cell comprising a primary cultured human fibroblasts.

48. Use of a primary cultured human fibroblasts as a feeder cell.

49. Use of a primary cultured human fibroblasts for producing a pharmaceutical composition containing a feeder cell.

50. Use of a primary cultured human fibroblasts for producing a pharmaceutical composition for regenerating an organ, tissue or cell of a subject.

51. A graft for regenerating an epithelial tissue which contains a stem cell or a cell derived from the stem cell.

52. The graft according to claim 51 wherein the epithelial tissue is cornea.

53. The graft according to claim 51 wherein the stem cell is selected from the group consisting of an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell and a hepatic stem cell.

54. The graft according to claim 51 wherein the stem cell is an epithelial stem cell.

55. The graft according to claim 51 wherein the stem cell is selected from the group consisting of a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, and a gallbladder epithelial stem cell.

56. The graft according to claim 51 wherein the stem cell or the cell derived from the stem cell has been co-cultured with a feeder cell.

57. The graft according to claim 56 wherein a human derived
cell is used as the feeder cell.

58. The graft according to claim 57 wherein the human derived cell comprises an adipose derived cell, an embryonic stem cell or a bone marrow stem cell.

59. The graft according to claim 58 wherein a cell cultured in DMEM+10% FBS in the absence of the feeder cell is used as the embryonic stem cell or bone marrow stem cell.

60. The graft according to claim 56 wherein the feeder cell comprises an adipose tissue derived cell.

61. The graft according to claim 56 wherein co-culturing with the feeder cell is performed under conditions that promote cell adhesion.

62. The graft according to claim 51 which comprises layered cells.

63. The graft according to claim 51 which is used in non-suture transplantation.

64. The graft according to claim 51 which does not contain any xenogeneic component.

65. Use for preparing a pharmaceutical composition as a graft for regenerating an epithelial tissue, the graft containing a stem cell or a cell derived from the stem cell.

66. The use according to claim 65 wherein the epithelial tissue is cornea.

67. The use according to claim 65 wherein the stem cell is selected from the group consisting of an epithelial stem cell, an embryonic stem cell, a bone marrow mesenchymal stem cell, a hematopoietic stem cell, a vascular endothelial stem cell, a neural stem cell, a retinal stem cell, an adipose stem cell, a renal stem cell and a hepatic stem cell.

68. The use according to claim 65 wherein the stem cell is an epithelial stem cell.

69. The use according to claim 65 wherein the stem cell is selected from the group consisting of a corneal epithelial stem cell, an oral mucosal epithelial stem cell, an epidermal stem cell, a bladder epithelial stem cell, a conjunctival epithelial stem cell, a gastric mucosal epithelial stem cell, a small intestine epithelial stem cell, a large intestine epithelial stem cell, a renal epithelial stem cell, a renal tubular epithelial stem cell, a gingival mucosal epithelial stem cell, a hair stem cell, an esophageal epithelial stem cell, a hepatic epithelial stem cell, a pancreatic epithelial stem cell, a mammary gland stem cell, a salivary gland stem cell, a lacrimal gland stem cell, a pulmonary epithelial stem cell, and a gallbladder epithelial stem cell.

70. The use according to claim 65 wherein the stem cell or the cell derived from the stem cell has been co-cultured with a feeder cell.


SEQUENCE LISTING

<110> NISHIDA, Kohji
      MAEDA, Kazuhisa

<120> REGENERATIVE MEDICAL TREATMENT SYSTEM

<130> ON001PCT

<160> 16

<170> PatentIn version 3.2

<210> 1
<211> 20
<212> DNA
<213> artificial

<220>
<223> primer

<400> 1
agaggacgtt tccaactcaa                                                20

<210> 2
<211> 20
<212> DNA
<213> artificial

<220>
<223> primer

<400> 2
tatgttccac aggtgacatc                                                20

<210> 3
<211> 20
<212> DNA
<213> artificial

<220>
<223> primer

<400> 3
aggaggatgg agatgctctg 20


<210> 4
<211> 20
<212> DNA
<213> artificial

<220>
<223> primer

<400> 4
tcagacttgc ggcaactctg 20


<210> 5
<211> 20
<212> DNA
<213> artificial

<220>
<223> primer

<400> 5
gcctgaaaga tatcccgaca 20

<210>  6
<211>  20
<212>  DNA
<213>  artificial

<220>
<223>  primcr

<400>  6
ttccatgttc ttgtcccaca                                                    20


<210>  7
<211>  20
<212>  DNA
<213>  artificial

<220>
<223>  primer

<400>  7
ggaggaatcc tgtgttgtta                                                    20


<210>  8
<211>  20
<212>  DNA
<213>  artificial

<220>
<223>  primer

<400>  8
ctaaggttca tgagcagtac                                                    20

<210> 9
<211> 19
<212> DNA
<213> artificial

<220>
<223> primer

<400> 9
cggagcgagg aagggaaag 19

<210> 10
<211> 23
<212> DNA
<213> artificial

<220>
<223> primer

<400> 10
ttggggataa actgcttgta ggc 23

<210> 11
<211> 17
<212> DNA
<213> artificial

<220>
<223> primer

<400> 11
atgcacacca tgtcctc 17

<210> 12
<211> 20
<212> DNA
<213> artificial

<220>
<223> primer

<400> 12
catcctgtag ttcttgtttc 20

<210> 13
<211> 20
<212> DNA
<213> artificial

<220>
<223> primer

<400> 13
accacagtcc atgccatcac 20

<210> 14
<211> 20
<212> DNA
<213> artificial

<220>
<223> primer

<400> 14
tccaccaccc tgttgctgta 20

<210> 15
<211> 20
<212> DNA
<213> artificial

<220>
<223> primer

<400> 15
tcctctctat ctagctccag 20

<210> 16
<211> 20
<212> DNA
<213> artificial

<220>
<223> primer

<400> 16
tcctgacagg tggatttcga 20